(19) Europäisches Patentamt / European Patent Office / Office européen des brevets

(11) **EP 3 663 760 A1**

(12) **EUROPEAN PATENT APPLICATION**

(43) Date of publication:
**10.06.2020 Bulletin 2020/24**

(51) Int Cl.:
**G01N 33/49** *(2006.01)*　**G01N 15/02** *(2006.01)*
**B01L 3/00** *(2006.01)*　**G01N 15/00** *(2006.01)*
**G01N 15/14** *(2006.01)*

(21) Application number: **19210591.4**

(22) Date of filing: **21.11.2019**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.11.2018   JP 2018226098**

(71) Applicant: **SYSMEX CORPORATION
Kobe-shi
Hyogo 651-0073 (JP)**

(72) Inventors:
• **Takahashi, Yusuke
  Hyogo, 651-0073 (JP)**
• **Shirai, Kentaro
  Hyogo, 651-0073 (JP)**
• **Yanagida, Masatoshi
  Hyogo, 651-0073 (JP)**
• **Iwanaga, Shigeki
  Hyogo, 651-0073 (JP)**
• **Ijiri, Yuichi
  Hyogo, 651-0073 (JP)**
• **Yoshikawa, Keiko
  Hyogo, 651-0073 (JP)**

(74) Representative: **Hoffmann Eitle
Patent- und Rechtsanwälte PartmbB
Arabellastraße 30
81925 München (DE)**

(54) **METHOD OF DETERMINING QUALITY OF CELL SEPARATION AND PARTICLE SEPARATION APPARATUS**

(57)　A method of determining a quality of a cell separation, according to an aspect, includes: separating, by running a specimen containing a first particle and a second particle in a flow path, the specimen to a first specimen containing the first particle and a second specimen containing the second particle, based on a difference in magnitude of a force exerted on each particle contained in the specimen; measuring first information on an amount of the first specimen and second information on an amount of the second specimen; and determining a quality of the separating based on the first information and the second information.

**EP 3 663 760 A1**

## Description

BACKGROUND

The present disclosure relates to a method of determining a quality of cell separation and a particle separation apparatus.

Cells are reflective of various conditions of a living body, such as the state of a subject's disease, and thus cells are measured for various purposes. To measure cells efficiently, it is desired to concentrate target cells. For example, in the case where rare cells in a sample are to be measured, such as circulating tumor cells (CTCs), circulating endothelial cells, hematopoietic stem cells in bone marrow fluid, and fetal erythroblasts contained in the blood of a pregnant woman, it is important to accurately separate the target cells from a large number of the other cells.

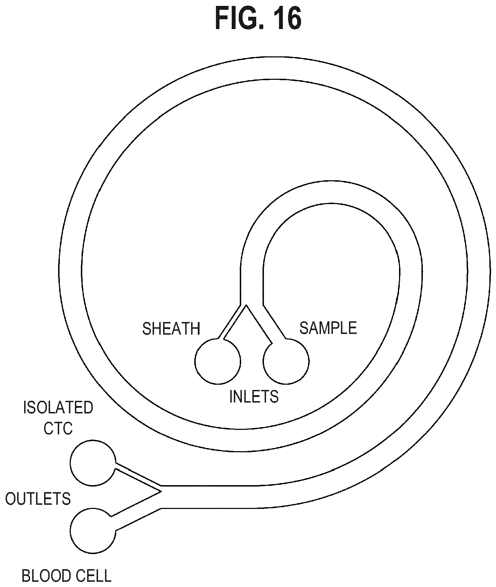

As a method of separating cells, Japanese National Publication of International Patent Application No. 2017-500006 ("Patent Document 1") discloses a method of isolating CTCs from the other cells by moving, along Dean vortexes, cells in a sample flowing in a curved flow path. A sample and sheath are fed into the inlets of the flow path illustrated in FIG. 16, and then, at the outlets, CTCs move close to the inner wall of the curved flow path, while RBCs and white blood cells move to the outer half of the channel. With this operation, the CTCs are separated and collected via the inner outlet.

In the case where a specimen is separated as described above and where the separated specimen is used in a clinical laboratory test, it is important to perform the separation step correctly. However, it is possible that the separation step is not performed properly because of factors such as, for example, bubbles generated in the flow path, clogging of the flow path resulting from the solidification of components in the blood that flows in the flow path, and a failure in a fluid mechanism of the separation apparatus connected to the chip including the flow path. In the case of grasping the symptom of a disease in a clinical laboratory test based on rare cells obtained by separating a specimen, if the number of the rare cells contained in the specimen after the separation is small because the separation step has not been performed correctly, a false negative may occur.

In light of this situation, an aspect aims to provide a method and an apparatus capable of determining a quality of a step of separating a specimen.

SUMMARY

A method of determining a quality of a cell separation, according to an aspect, includes: separating, by running a specimen containing a first particle and a second particle in a flow path, the specimen to a first specimen containing the first particle and a second specimen containing the second particle, based on a difference in magnitude of a force exerted on each particle contained in the specimen; measuring first information on an amount of the first specimen and second information on an amount of the second specimen; and determining a quality of the separating based on the first information and the second information.

A particle separation apparatus according to an aspect includes: a separation unit that separates, by running a specimen in a flow path, the specimen to a first specimen and a second specimen, based on a difference in magnitude of a force exerted on each particle contained in the specimen; a measurement unit that measures first information on an amount of the first specimen and second information on an amount of the second specimen; and a determination unit that determines a quality of the separation of the specimen by the separation unit based on the first information and the second information.

BRIEF DESCRIPTION OF DRAWINGS

FIG. 1 is a flow diagram illustrating steps of a method of determining whether a separation is successful according to an embodiment;
FIG. 2 is a schematic diagram illustrating a separation step according to an embodiment;
FIG. 3A is a schematic diagram illustrating a configuration of a chip according to an embodiment;
FIG. 3B is a schematic diagram illustrating a cross-sectional view of a micro flow path according to an embodiment;
FIG. 4A is a schematic diagram illustrating an end portion on an output end portion side of a micro flow path according to an embodiment;
FIG. 4B is a schematic diagram illustrating a measurement step according to an embodiment;
FIG. 5 is a flow diagram illustrating steps of a method of determining whether a separation is successful according to a modification example of an embodiment;
FIG. 6A is a diagram illustrating a scatter plot drawn in a study of a method of determining whether a separation is successful according to an embodiment;
Fig. 6B is a diagram illustrating a scatter plot drawn in verification of a method of determining whether a separation is successful according to an embodiment;
FIG. 7 is a flow diagram illustrating steps of a particle detection method according to an embodiment;
FIG. 8 is a schematic diagram illustrating a staining step according to an embodiment;
FIG. 9 is a schematic diagram illustrating a concentration step according to an embodiment;
FIG. 10 is a schematic diagram illustrating a detection step according to an embodiment;
FIG. 11 is a schematic diagram illustrating a configuration of a particle detection system according to an embodiment;

FIG. 12 is a schematic diagram illustrating a configuration of a particle separation apparatus according to an embodiment;

FIGs. 13A and 13B are schematic diagrams illustrating screens including determination results, displayed on a display-input unit of a particle separation apparatus according to an embodiment;

FIG. 14 is a schematic diagram illustrating a configuration of a staining-concentration apparatus according to an embodiment;

FIG. 15 is a schematic diagram illustrating a configuration of a flow cytometer according to an embodiment; and

FIG. 16 is a schematic diagram illustrating a configuration according to a related art.

## DETAILED DESCRIPTION

A first aspect relates to a method of determining a quality of a cell separation. The method according to an aspect includes: a separation step (S1) of separating, by running a specimen (21) containing a first particle and a second particle in a flow path (110), the specimen (21) to a first specimen (22) containing the first particle and a second specimen (23) containing the second particle, based on a difference in the magnitude of a force exerted on each particle contained in the specimen (21); a measurement step (S2) of measuring first information on an amount of the first specimen (22) and second information on an amount of the second specimen (23); and a determination step (S3) of determining a quality of the separation step (S1) based on the first information and the second information.

An amount of the first specimen means the weight or volume of the first specimen, and an amount of the second specimen means the weight or volume of the second specimen. The first information means information that enables calculation of the amount of the first specimen. Examples of the first information include the value of the amount of the first specimen measured and the flow speed of the first specimen which can be used for calculation of the amount of the first information. The second information means information that enables calculation of the amount of the second specimen. Examples of the second information include the value of the amount of the second specimen measured and the flow speed of the second specimen which can be used for calculation of the amount of the second information.

The inventors have found that the state of separation of the first specimen and the second specimen is reflected on the first information on the amount of the first specimen and the second information on the amount of the second specimen. Hence, use of the method according to an aspect makes it possible to determine a quality of the separation step, such as whether the separation step is successful or not, based on the first information and the second information measured at the measurement step.

Since whether the quality of the separation step can be determined in this way, it is possible to supply the first specimen to the subsequent steps only in the case where the specimen has been properly separated to the first specimen and the second specimen. Since the first specimen is supplied to the subsequent steps only in the case where the first specimen has been properly separated from the specimen, the number of first particles contained in the measurement specimen supplied to the subsequent detection step can be made closer to the number of first particles contained in the specimen before the separation. Thus, for example, in the case where the first particles are rare cells and the state of the disease is judged based on the detection result of the first particles, the first particles are properly separated from a specimen, and the detection is performed on a measurement specimen containing enough first particles. This process prevents a false negative in judging the state of the disease, which would be caused in the case where the rare cells are lost in separation, and the number of rare cells contained in the specimen after the separation is small.

For the method of determining a quality of a separation, according to an aspect, in the separation step (S1), the specimen (21) is separated to the first specimen (22) in which a presence ratio of the first particle is higher than in the specimen (21) and the second specimen (23) in which a presence ratio of the first particle is lower than in the specimen (21). The presence ratio here means (the number of first particles in a specimen)/(the number of particles of specified kinds in the specimen). For example, in the case where the first particles are circulating tumor cells (CTCs), the presence ratio is (the number of CTCs in a specimen)/(the number of blood cells in the specimen).

In method of determining a quality of a separation, according to an aspect, the difference in the magnitude of the force exerted on each particle contained in the specimen is caused by a difference in a property of the particle.

In this case, the difference in the property of the particle includes at least one of a difference in size, a difference in weight, or a difference in specific gravity, such as at least one of: the difference in size; the difference in weight; and the difference in specific gravity.

For the method of determining a quality of a separation, according to an aspect, in the separation step (S1), the specimen (21) is separated to the first specimen (22) and the second specimen (23) by running the specimen (21) in a micro flow path (110). This method makes it possible to downsize the configuration for performing the separation step.

For the method of determining a quality of a separation, according to an aspect, in the separation step (S1), the specimen (21) is separated to the first specimen (22) and the second specimen (23) by running the specimen (21) in the flow path (110) including a curved section (111). This method makes it possible to separate the specimen to the first specimen and the second specimen with a simple configuration and in an effective manner.

For method of determining a quality of a separation, according to an aspect, in the separation step (S1), the specimen (21) is separated to the first specimen (22) and the second specimen (23) based on a speed distribution in the flow path (110). This method makes it possible to separate a specimen to a first specimen and a second specimen by just running the specimen in the micro flow path.

For the method of determining a quality of a separation, according to an aspect, in the separation step (S1), the specimen (21) containing a sample and sheath liquid is run in the flow path (110), and the first specimen (22) and the second specimen (23) contain the sample and the sheath liquid.

In the method of determining a quality of a separation, according to an aspect, the measurement step (S2) is performed after the separation step (S1). Since the first information and the second information on which the state after the separation step is reflected are obtained in this operation, it is possible to determine the quality of the separation step in the determination step.

For the method of determining a quality of a separation, according to an aspect, in the measurement step (S2), the first information and the second information are measured based on a detection signal of a detector (151, 152) provided on each of a first flow path (141) in which the first specimen (22) flows and a second flow path (142) in which the second specimen (23) flows. Use of the detection signal of the detector makes it possible to obtain the flow speed of the first specimen flowing in the first flow path as the first information and obtain the flow speed of the second specimen flowing in the second flow path as the second information. The obtained first information, which here is the flow speed, is information that enables calculation of an amount of the first specimen, and the obtained second information, which here is the flow speed, is information that enables calculation of an amount of the second specimen. Thus, a quality of the separation step, such as whether the separation step is successful or not, can be determined based on the first information and the second information.

In the method of determining a quality of a separation, according to an aspect, the first information is on the weight or volume of the first specimen (22), and the second information is on the weight or volume of the second specimen (23).

In the method of determining a quality of a separation, according to an aspect, the first information and the second information can be information that enables calculation of the weight or volume of the first specimen (22) and the second specimen (23), respectively.

In the method of determining a quality of a separation, according to an aspect, the first information and the second information can be on pressure or flow speed.

For the method of determining a quality of a separation, according to an aspect, in the determination step (S3), a quality of the separation step (S1) is determined based on a ratio calculated from the first information and the second information. The inventors have found that the state of separation of the first specimen and the second specimen is reflected on a ratio calculated from the first information and the second information. Hence, the quality of the separation step can be determined based on the calculated ratio.

In this case, the ratio can be the ratio of the sum of the first information and the second information to the first information, the ratio of the sum of the first information and the second information to the second information, the ratio of the first information to the sum of the first information and the second information, the ratio of the second information to the sum of the first information and the second information, the ratio of the second information to the first information, or the ratio of the first information to the second information.

For the method of determining a quality of a separation, according to an aspect, in the determination step (S3), a quality of the separation step (S1) is determined by comparing the ratio and a specified threshold.

For the method of determining a quality of a separation, according to an aspect, in the determination step (S3), a quality of the separation step (S1) is determined based on whether the ratio is higher than a specified threshold or based on whether the ratio is lower than a specified threshold. With this method, in the case where the number of the particles corresponding to the first particles is extremely large or extremely small in the first specimen, it can be determined that the separation step has not been performed correctly.

For the method of determining a quality of a separation, according to an aspect, in the determination step (S3), a quality of the separation step (S1) is determined based on whether the ratio is within a specified range. With this method, in the case where the number of the particles corresponding to the first particles is extremely large or extremely small in the first specimen, it can be determined that the separation step has not been performed correctly

For the method of determining a quality of a separation, according to an aspect, in the determination step (S3), a quality of the separation step (S1) is determined based on the sum of the first information and the second information. In the case where a failure has occurred in the separation step, it is possible that amounts concerning the first specimen and second specimen obtained are decreased. In this case, since appropriate amounts concerning the first specimen and second specimen are not obtained, the separation step is not performed correctly. Thus, by determining, in the determination step, whether the sum of the first information on the amount of the first specimen and the second information on the amount of the second specimen is larger than a specified threshold, it is possible to further determine a quality of the separation step.

For the method of determining a quality of a separation, according to an aspect, in a case where it is determined in the determination step (S3) that the separation step (S1) has not been performed correctly, the separation step (S1) is performed again. With this method, in the case where the separation step has not been performed correctly, it prevents an improper first specimen from being supplied to the subsequent process and thus makes it possible to resume the separation step smoothly to obtain a proper first specimen.

In the method of determining a quality of a separation, according to an aspect, the specimen (21) includes blood.

In the method of determining a quality of a separation, according to an aspect, the first particle is a circulating tumor cell.

In the method of determining a quality of a separation, according to an aspect, the second particle includes at least one kind of blood cell selected from a red blood cell, a white blood cell, or a platelet, such as at least one kind of blood cell of: a red blood cell; a white blood cell; and a platelet.

A second aspect relates to particle detection methods. A particle detection method according to an aspect includes: measuring, with a flow cytometer (13) or a microscope, the first specimen (22) for which the separation step (S1) has been determined to have been performed correctly in the method of determining a quality of a separation according to a first aspect; and detecting the first particle contained in the specimen (21).

In the particle detection method according to an aspect, only in the case where the separation step has been determined to have been performed correctly, the first particle is detected with a flow cytometer or a microscope based on the first specimen. With this method, the number of the first particles contained in the measurement specimen supplied to a flow cytometer or a microscope can be made closer to the number of the first particles contained in the specimen before separation. Thus, for example, in the case where the first particles are rare cells and where the state of a disease is judged based on the detection result of the rare cells, the detection is performed on a measurement specimen that is properly separated from a specimen and contains enough first particles. Thus, it is possible to prevent a false negative in judgment of the state of a disease, which would be caused in the case where the number of rare cells contained in a specimen after separation is small.

A third aspect relates to particle separation apparatuses. A particle separation apparatus (11) according to an aspect includes: a separation unit (104) that separates, by running a specimen (21) in a flow path (110), the specimen (21) to a first specimen (22) and a second specimen (23), based on a difference in the magnitude of a force exerted on each particle contained in the specimen (21); a measurement unit (105) that measures first information on an amount of the first specimen (22) and second information on an amount of the second specimen (23); and a determination unit (controller, 101) that determines a quality of the separation of the specimen (21) by the separation unit (104) based on the first information and the second information.

The particle separation apparatus according to an aspect provides the same effects as a first aspect does.

In the particle separation apparatus (11) according to an aspect, the separation unit (104) includes a pump (104a) that runs the specimen (21) in the flow path (110).

In the particle separation apparatus (11) according to an aspect, the flow path (110) can be formed in a replaceable chip (100). This configuration makes it possible to separate a different specimen by just replacing the chip without troublesome works such as cleaning the flow path.

In the particle separation apparatus (11) according to an aspect, the flow path (110) can include a curved section (111). This makes it possible to separate the specimen to the first specimen and the second specimen with a simple configuration and in an effective manner.

The particle separation apparatus (11) according to an aspect can further include a display unit (103) that displays a determination result by the determination unit (controller, 101). With this configuration, an operator can visually realize whether a separation of the specimen has been performed properly.

An aspect makes it possible to determine a quality of the separation step, such as whether the separation step is successful or not.

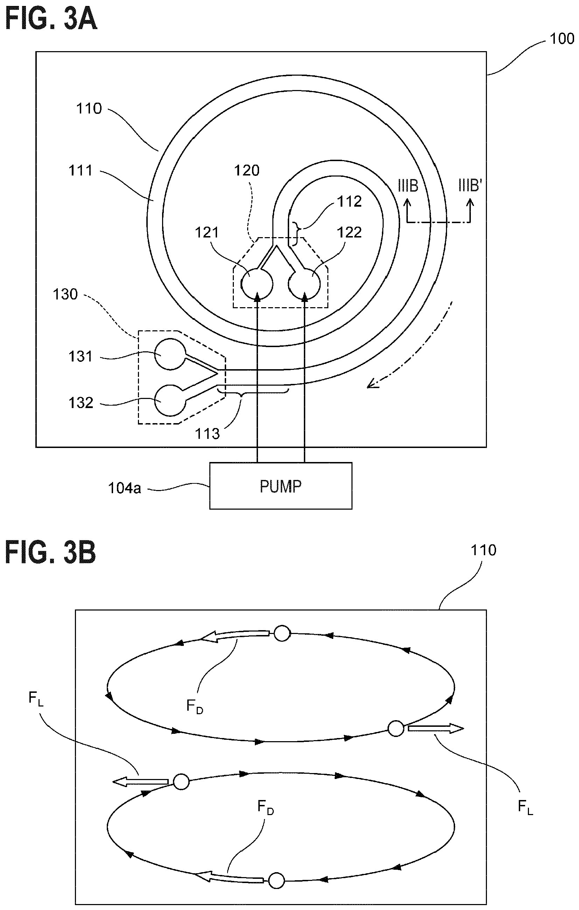

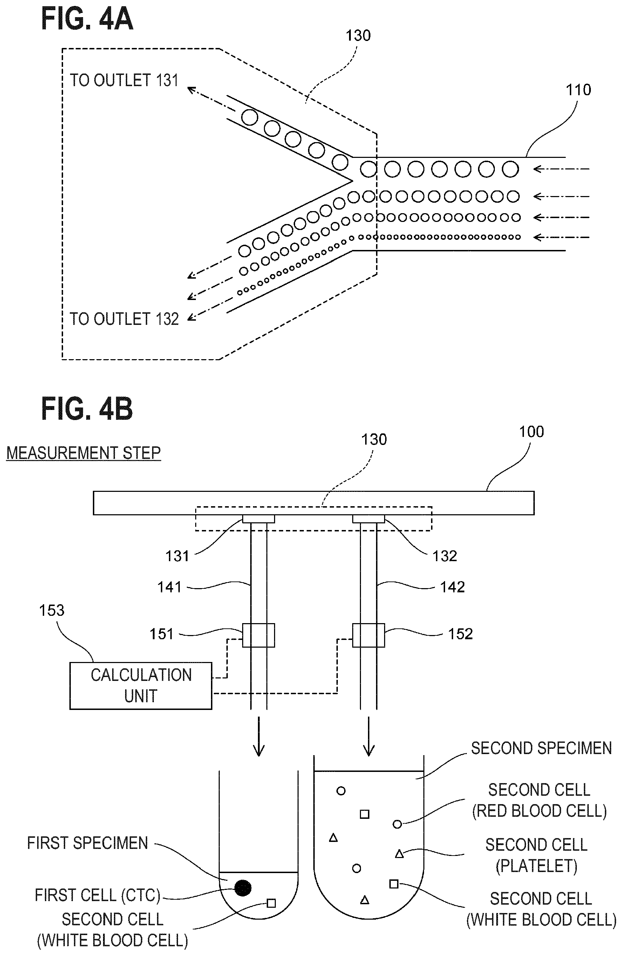

A method of determining a quality of a separation, a particle detection method, and a particle separation apparatus according to an embodiment described below are for running a specimen containing first particles and second particles in a flow path and for separating the specimen to a first specimen containing the first particles and a second specimen containing the second particles based on the difference in the magnitude of a force exerted on each particle contained in the specimen. Specifically, a specimen is run in a micro flow path 110 of a chip 100 illustrated in FIG. 3A, the magnitude of the force exerted on each particle is made different in the micro flow path 110 according to the diameter of the particle, and thereby the position where the particle flows is made different as illustrated in FIG. 3B. Thus, the specimen is separated, and a first specimen and a second specimen are obtained from outlets 131 and 132 illustrated in FIG. 3A, respectively. Then, first information on an amount of the first specimen obtained from the separation and second information on an amount of the second specimen obtained from the separation are measured. On the basis of the first information and the second information, whether the separation step is successful is determined. Specifically, using pressure detectors 151 and 152 illustrated in FIG. 4B, the flow speed of the first specimen flowing out of the outlet 131 is obtained as the first information, and the flow speed of the second specimen flowing out of the outlet 132 is obtained as the second information. After that, a ratio based on the first information and the second information is calculated, and based on the calculated ratio, whether the separation step is successful is determined.

An example in which a specimen contains blood is described below according to an embodiment. However, the specimen is not limited to blood, but, for example, it may be urine, bone marrow fluid, or the like. An embodiment is described below based on an example in which first particles are circulating tumor cells (CTCs), and second particles are white blood cells.

The first particles are not limited to CTCs but may be cells other than CTCs. The first particles may be circulating endothelial cells (CECs) or hematopoietic stem cells (HSCs). The first particles may be nanovesicles, microvesicles, red blood cells, white blood cells, platelets, particles contained in plasma, or particles contained in serum. Nanovesicles are, for example, exosomes. The particles contained in plasma and the particles contained in serum are, for example, proteins or fine particles. The first particles may be abnormal lymphocyte cells.

<Method of Determining Whether a separation is Successful>

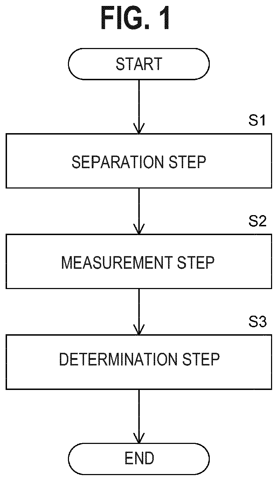

FIG. 1 is a flowchart illustrating steps of a method of determining whether a separation is successful.

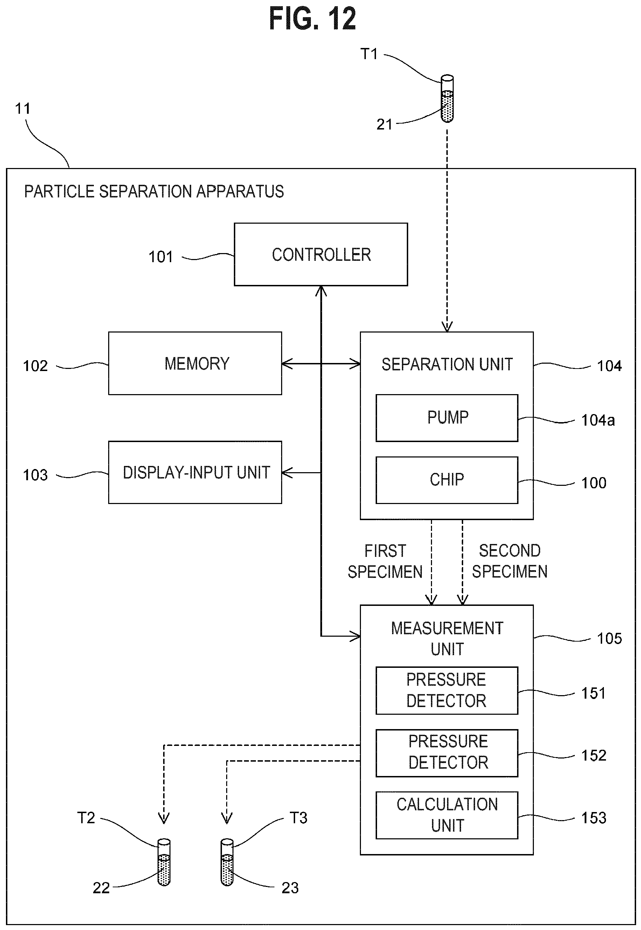

A method of determining whether a separation is successful in an embodiment includes a separation step (step S1) a measurement step (step S2) and a determination step (step S3). Hereinafter, description is made of the case that an operator inputs an instruction to a particle separation apparatus in each step, the particle separation apparatus executes each step according to the inputted instruction. Note that all the steps S1 to S3 may be performed automatically by the particle separation apparatus or may be performed by the operator operating the apparatus. The particle separation apparatus is described later with reference to FIG. 12.

In the following description, first particles are particles to be detected in a detection step described later and to be analyzed in an analysis step described later, and hence the first particles are target particles. The first particles, specifically CTCs, are referred to as the "first cells." The second particles, specifically red blood cells, white blood cell, platelets, and the like, are referred to as the "second cells."

In the separation step at step S1, the particle separation apparatus separates a specimen containing the first cells and the second cells to a first specimen containing the first cells and a second specimen containing the second cells. To be more specific, in the separation step at step S1, the particle separation apparatus runs the specimen containing the first cells and the second cells in a flow path. Then, the particle separation apparatus separates the specimen to the first specimen and the second specimen based on the difference in the magnitude of the force exerted on the particles contained in the specimen. In this operation, the particle separation apparatus separates the specimen to the first specimen with a higher presence ratio of the first cells than the specimen and the second specimen with a lower presence ratio of the first cells than the specimen.

The difference in the magnitude of the force exerted on each particle contained in the specimen is caused by the difference in the properties of the particle. The properties of the particle in this case mean morphological properties and physical properties. An example of morphological properties is the size of a particle. The size of a particle means, for example, the diameter or radius, the area in a plan view, the length of the perimeter, and the like of the particle. Examples of the physical properties include weight and specific gravity.

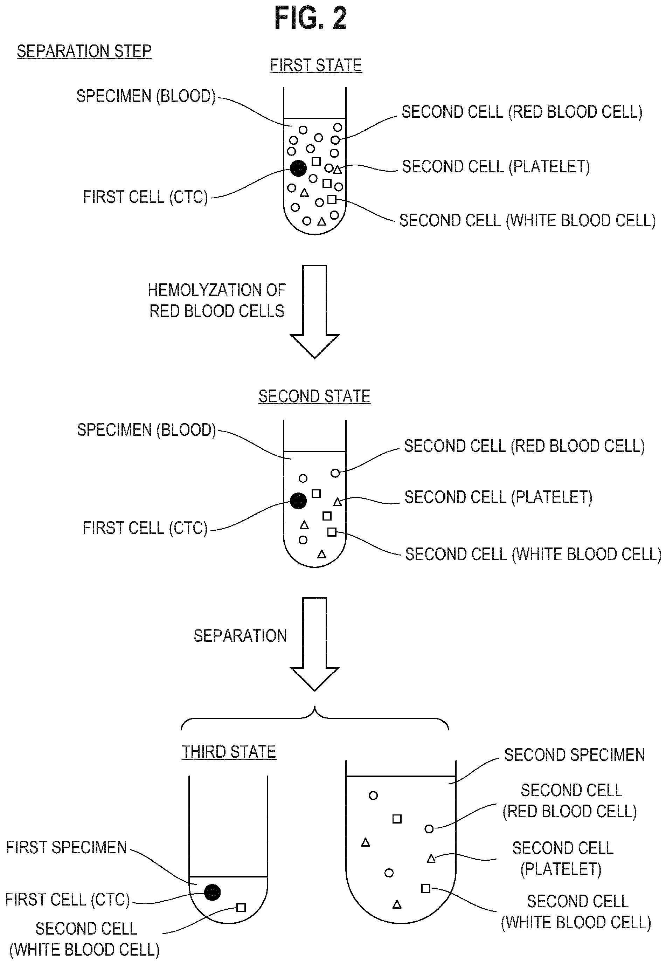

FIG. 2 is a schematic diagram for explaining the separation step at step S1.

In the specimen to be supplied to the particle separation apparatus, the red blood cells have been hemolyzed beforehand. A specimen just after it is collected from a subject is blood as illustrated in the first state, specifically, whole blood. The specimen in the first state contains CTCs as first cells and red blood cells, platelets, and white blood cells as second cells. In FIG. 2, a CTC is represented by a black dot, red blood cells are represented by circles, platelets are represented by triangles, and white blood cells are represented by squares. In the specimen in the first state, the operator hemolyzes the red blood cells using a specified reagent and then centrifuges the specimen with a centrifuge to remove the supernatant. With this operation, most of the red blood cells have been removed from the specimen in the first state, and the resultant thus obtained is referred to as the specimen in the second state. Then, the specimen in the second state is supplied to the particle separation apparatus.

Note that the particle separation apparatus may perform a process to change the specimen from the first state to the second state. For example, the particle separation apparatus may include a mechanism for hemolyzing red blood cells, a centrifuge, and the like. Alternatively, for example, in the separation step at step S1, a process for changing the specimen from the first state to the second state may be performed by separating red blood cells from the other particles with the chip 100 described later with reference to FIG. 3A, by utilizing the difference in the morphological properties and physical properties of the particles. Alternatively, the separation step may be performed omitting the process of changing the specimen from the first state to the second state and performed using the specimen in the first state as the specimen in the second state.

At step S1, the particle separation apparatus separates the first cells and the second cells from each other in the specimen in the second state. Specifically, on the basis of the difference in the magnitude of the force exerted on each particle between CTCs, red blood cells, platelets, and white blood cells, the particle separation apparatus separates and removes the red blood cells, platelets, and white blood cells from the specimen in the second state. In an embodiment, taking advantage of the fact that the difference in the magnitude of the force exerted on cells is caused by the difference in diameter between the cells, the separation is performed by running the specimen in the flow path. With this operation, the specimen in the second state is separated to the first specimen containing the first cells and the second specimen containing the second cells. Thus, the first specimen containing the first cells is obtained as illustrated in the third state.

As described above, in the case where red blood cells are hemolyzed and removed from the blood in the first state before the specimen is separated, red blood cells which are contained in blood in a large amount have been removed beforehand in the specimen for the separation step. This operation makes it smooth to separate the specimen to the first specimen and the second specimen in the separation of the specimen.

When the specimen in the second state illustrated in FIG. 2 is separated to the first specimen and the second specimen, the chip 100 illustrated in FIG. 3A is used.

FIG. 3A is a schematic diagram illustrating the configuration of the chip 100.

The specimen is run in the micro flow path 110 formed in the chip 100, and thereby the chip 100 separates the specimen to the first specimen and the second specimen. In this operation, the chip 100 separates the specimen to the first specimen and the second specimen such that the presence ratio of the first particles in the first specimen is higher than the presence ratio of the first particles in the specimen. Note that the presence ratio here means: (the number of first particles in the specimen)/(the number of particles of specified kinds in the specimen). In the case where the first particles are CTCs, the presence ratio is, for example, (the number of CTCs in the specimen)/(the number of blood cells in the specimen) or (the number of CTCs in the specimen)/(the number of white blood cells in the specimen). The number of particles of specified kinds in the specimen, which is the denominator of the presence ratio, may be the number of any kinds of particles as long as a certain kind of particles unnecessary in the measurement of the first particle are removed even partially from the specimen after the separation step. In the case where the first particles are exosomes, the presence ratio is, for example, (the number of exosomes particles in the specimen)/(the number of blood cells in the specimen). In the case where the first particles are protein contained in plasma, the presence ratio is, for example, (the number of protein molecules in the specimen)/(the number of blood cells in the specimen).

The chip 100 includes the micro flow path 110, an input end portion 120, and an output end portion 130.

The micro flow path generally means a flow path with a depth and width in the range of 10 $\mu$m to 1000 $\mu$m. The chip 100 is formed, for example, by placing a plate member on a thin plate member including grooves formed corresponding to the micro flow path 110, the input end portion 120, and the output end portion 130. Separation of a specimen in an embodiment is based on a separation principle called Dean Flow Fractionation.

The micro flow path 110 includes a curved section 111 in a spiral shape and straight sections 112 and 113. The shape of the curved section 111 is an arc shape as viewed from a direction perpendicular to the chip 100. The shapes of the straight sections 112 and 113 are linear as viewed from a direction perpendicular to the chip 100. The input end portion 120 is formed at the end portion on the center side of the spiral shape of the micro flow path 110, and the output end portion 130 is formed at the end portion opposite from the input end portion 120 of the micro flow path 110. The input end portion 120 is connected to the curved section 111 via the straight section 112, and the output end portion 130 is connected to the curved section 111 via the straight section 113.

The input end portion 120 includes inlets 121 and 122. The inlets 121 and 122 are holes formed in the member of the chip 100. The inlet 121 is connected to the curved section 111 via the straight section 112 on the outer periphery side of the spiral shape of the micro flow path 110. The inlet 122 is connected to the curved section 111 via the straight section 112 on the inner periphery side of the spiral shape of the micro flow path 110. The output end portion 130 includes the outlets 131 and 132. The outlets 131 and 132 are holes formed in the member of the chip 100. The outlet 131 is connected to the curved section 111 via the straight section 113 on the inner periphery side of the spiral shape of the micro flow path 110. The outlet 132 is connected to the curved section 111 via the straight section 113 on the outer periphery side of the spiral shape of the micro flow path 110.

To separate a specimen, a specimen in the second state illustrated in FIG. 2 is infused into the inlet 121, and sheath liquid is infused into the inlet 122. The amount of the specimen in the second state infused into the inlet 121 is, for example, 5 mL, and the amount of the sheath liquid infused into the inlet 122 is, for example, 45 mL. Then, a pump 104a connected to the inlets 121 and 122 via tubes applies positive pressure to the inlets 121 and 122. This makes the specimen infused into the inlet 121 and the sheath liquid infused into the inlet 122 flow within the micro flow path 110 toward the output end portion 130. The specimen and sheath liquid are run in the micro flow path 110, and thus both the first specimen obtained from the outlet 131 and the second specimen obtained from the outlet 132 contain the specimen and sheath liquid. The amount of the first specimen obtained from the outlet 131 is, for example, 10 mL, and the amount of the second specimen obtained from the outlet 132 is, for example, 40 mL.

FIG. 3B is a cross-sectional view taken along line IIIB-IIIB' of the micro flow path 110 illustrated in FIG. 3A. In FIG. 3B, the right side is the outer periphery side of the spiral shape, and the left side is the inner periphery side of the spiral shape.

When the specimen in the second state is run in the micro flow path 110, the force that changes the positions of the flows of cells is generated in a direction orthogonal to the direction of the flow path in the micro flow path 110, as illustrated in FIG. 3B. Specifically, lift $F_L$ and Dean drag $F_D$ having different magnitudes according to the diameter of each cell are generated. Generally, in the distribution of the flow speed in the micro flow path, the flow speed around the center of the section of the flow path is high, and the flow speed near the walls is low. Further, in the micro flow path 110 in a spiral shape, secondary flows called Dean vortexes are generated resulting from the above flow speed distribution, as indicated by the elliptical arrows in FIG. 3B. Dean drag $F_D$ and lift $F_L$ are expressed by the following formulae (1) and (2).

[Math. 1]

$$F_D = 3\pi\mu\overline{U}_{Dean}a_p \qquad \cdots (1)$$

$$F_L = \rho G^2 C_L a_p^{\,4} \qquad \cdots (2)$$

$\mu$: the viscosity of the fluid
$\rho$: the density of the fluid
$\overline{U}_{Dean}$: Dean velocity
$G$: shear rate
$a_p$: the diameter of the particle
$C_L$: lift coefficient

$$G = U_{max}/D_h$$

$U_{max}$: the maximum velocity in the micro flow path
$D_h$: hydraulic diameter

As the cells in the specimen are pushed and run along the micro flow path 110, the cells get distributed within the micro flow path 110 according to the diameter of each cell because of lift $F_L$ and Dean drag $F_D$.

FIG. 4A is a schematic diagram illustrating the end portion on the output end portion 130 side of the micro flow path 110.

When Dean vortexes are generated in the micro flow path 110, the cells get distributed according to the respective diameters at the end portion on the output end portion 130 side of the micro flow path 110, as illustrated in FIG. 4A. Thus, cells with diameters of a specified value or more are obtained from the outlet 131. The specimen obtained from the outlet 131 is the first specimen in the third state in FIG. 2, and the specimen obtained from the outlet 132 is the second specimen in FIG. 2.

The diameters of most white blood cells are values around 10 μm to 15 μm or less, and the diameters of most cancer cells are values around 10 μm to 15 μm or more. Hence, to remove the second cells from the second state in FIG. 2, the cut-off value should preferably be around 10 μm to 15 μm, and the cells with diameters of the cut-off value or larger should preferably be taken out by removing the cells with diameters smaller than the cut-off value.

The use of the micro flow path 110 as above makes it possible to separate a specimen to a first specimen and a second specimen with a simple configuration, and thus achieve downsizing of the structure for the separation step. Since the separation is performed by running a specimen in the micro flow path 110 with the curved section 111, the separation of the specimen to the first specimen and the second specimen is possible with a simple configuration and in an effective manner. Since the separation performed in the micro flow path 110 is based on the speed distribution in the curved section 111, it is possible to separate a specimen to a first specimen and a second specimen by just running the specimen in the micro flow path 110.

Note that the micro flow path 110 is also capable of separating plasma, as a first specimen, from a specimen. In this case, protein and fine particles contained in plasma are first particles. Because the diameters of the protein and fine particles, which are the first particles, are small, the first particles are led to the outlet 132, and plasma is obtained from the outlet 132.

Returning to FIG. 1, in the measurement step at step S2, the particle separation apparatus measures first information on an amount of the first specimen and second information on an amount of the second specimen, based on the first specimen and the second specimen obtained in the separation step.

An amount of the first specimen means the weight or volume of the first specimen, and an amount of the second specimen means the weight or volume of the second specimen. The first information is information that enables calculation of the amount of the first specimen. Examples of the first information include the value directly indicating the amount of the first specimen, the flow speed of the first specimen which can be used for calculation of the amount of the first specimen, and the pressure of the flow path in which the first specimen is flowing. The second information is information that enables calculation of the amount of the second specimen. Examples of the second information include the value directly indicating the amount of the second specimen, the flow speed of the second specimen which can be used for calculation of the amount of the second specimen, and the pressure of the flow path in which the second specimen is flowing. In an embodiment, the amount of the first specimen is the weight of the first specimen, and the first information is the flow speed of the first specimen. The amount of the second specimen is the weight of the second specimen, and the second information is the flow speed of the second specimen.

FIG. 4B is a schematic diagram for explaining the measurement step at step S2.

The outlet 131 of the chip 100 is connected to a first flow path 141. The outlet 131 is connected to a container for storing the first specimen via the first flow path 141. The first flow path 141 leads the first specimen that flows out of the outlet 131 to the container for storing the first specimen. The first flow path 141 is a tube. Similarly, the outlet 132 of the chip 100 is connected to a second flow path 142. The outlet 132 is connected to a container for storing the second specimen via the second flow path 142. The second flow path 142 leads the second specimen that flows out of the outlet 132 to the container for storing the second specimen. The second flow path 142 is a tube.

The pressure detector 151 is provided at the first flow path 141 for measuring the flow speed of the first specimen flowing in the first flow path 141. The pressure detector 151 detects the pressure within the first flow path 141. Similarly, the pressure detector 152 is provided at the second flow path 142 for measuring the flow speed of the second specimen flowing in the second flow path 142. The pressure detector 152 detects the pressure within the second flow path 142. The pressure detectors 151 and 152 are connected to a calculation unit 153. The calculation unit 153 obtains the flow speed inside the first flow path 141 and the flow speed inside the second flow path 142 based on detection signals of the pressure detectors 151 and 152.

Note that instead of the pressure detectors 151 and 152, a flow speed detector that detects the pressure within the first flow path 141 to obtain the flow speed and a flow speed detector that detects the pressure within the second flow path 142 to obtain the flow speed may be provided.

Here, the measured flow speed of the first specimen is information that enables calculation of the weight of the first specimen. Specifically, the weight of the first specimen can be calculated by multiplying together the flow speed of the first specimen in the first flow path 141, the cross-sectional area of the first flow path 141, the period of time for which the first specimen has flowed in the first flow path 141, and the specific gravity of the first specimen. Similarly, the measured flow speed of the second specimen is information that enables calculation of the weight of the second specimen. Specifically, the weight of the second specimen can be calculated by multiplying together the flow speed of the second specimen in the second flow path 142, the cross-sectional area of the second flow path 142, the period of time for which the second specimen has flowed in the second flow path 142, and the specific gravity of the second specimen.

Note that the measured flow speed of the first specimen can be used not only for calculating the weight but for calculating the volume of the first specimen by multiplying together the measured flow speed of the first specimen, the cross-sectional area of the first flow path 141, and the period of time for which the first specimen has flowed in the first flow path 141. Similarly, the measured flow speed of the second specimen is information that enables calculation of the volume of the second specimen.

The detection signal of the pressure detector 151 is used to obtain the flow speed of the first specimen flowing in the first flow path 141 as the first information. The detection signal of the pressure detector 152 is used to obtain the flow speed of the second specimen flowing in the second flow path 142 as the second information. The obtained first information, which here is the flow speed, is information that enables calculation of the amount of the first specimen. The obtained second information, which here is the flow speed, is information that enables calculation of the amount of the second specimen. Thus, on the basis of the first information and the second information, whether the separation step is successful can be determined in the subsequent determination step.

The measurement step described above is performed after the separation step. Specifically, the separation is finished in the chip 100, and then the flow speeds of the first specimen and the second specimen flowing out of the outlets 131 and 132 are measured. Since the state after the separation step is reflected on the first information and the second information obtained in this operation, it is possible to determine whether the separation step is successful in the determination step.

Returning to FIG. 1, in the determination step at step S3, the particle separation apparatus determines whether the separation step at step S1 is successful based on the first information and the second information.

Meanwhile, the inventors have found that the state of separation of the first specimen and the second specimen is reflected on the first information on the amount of the first specimen containing the first cells and the second information on the amount of the second specimen containing the second cells. Hence, whether the separation step at step S1 is successful can be determined in the determination step at step S3 based on the first information and the second information measured in the measurement step at step S2. To be more specific, the inventors have found that the state of separation of the first specimen and the second specimen is reflected on a ratio calculated from the first information and the second information. Hence, whether the separation step is successful can be determined based on the ratio calculated from the first information and the second information. Note that the fact that the state of separation is reflected on the ratio calculated from the first information and the second information is explained later along with the study that the inventors have conducted.

In an embodiment, the ratio calculated from the first information and the second information is the ratio of the sum of the first information and the second information to the first information. In other words, the ratio in an embodiment is: (the first information + the second information)/the first information.

Note that the calculated ratio may be the ratio of the sum of the first information and the second information to the second information, the ratio of the first information to the sum of the first information and the second information, the ratio of the second information to the sum of the first information and the second information, or the like. In other words, the ratio may be: (the first information + the second information)/the second information, the first information/(the first information + the second information), or the second information/(the first information + the second information). The calculated ratio may be the ratio of the second information to the first information, the ratio of the first information to the second information, or the like. In other words, the ratio may be: the second information/the first information, or the first information/the second information.

In the determination step at step S3, the particle separation apparatus determines whether the separation step is successful based on whether the ratio calculated as described above is higher than a specified threshold or whether the ratio calculated as described above is lower than the specified threshold. It depends on the calculation formula of the ratio whether: the separation step is determined to have been performed correctly in the case that the ratio is higher than the specified threshold; or the separation step is determined to have been performed correctly in the case that the ratio is lower than the specified threshold. Determination as described above makes it possible to determine that the separation step has not been performed correctly in the case where the number of particles corresponding to the first cells is extremely large or extremely small in the first specimen.

Note that the particle separation apparatus may determine whether the separation step is successful based on whether the ratio calculated as described above is within a specified range. With such determination, it is possible to determine that the separation step has not been performed correctly in the case where the number of particles corresponding to the first cells is extremely large or extremely small in the first specimen.

Meanwhile, examples of factors that prevent a correct separation step include bubbles generated in the micro flow path 110, clogging of the micro flow path 110 resulting from the solidification of components in the blood that flows in the micro flow path 110, molding defects of the micro flow path 110, and a failure in the fluid mechanism of the particle separation apparatus connected to the chip 100. However, in the case where the measurement step and the determination step are performed as described above, it is possible to realize that the separation step has not been appropriately performed due to these factors.

If whether the separation step is successful can be determined as described above, only in the case where a specimen has been properly separated to a first specimen and a second specimen, the first specimen can be supplied to the subsequent process. If the first specimen is supplied to the subsequent steps only in the case where the first specimen is properly separated from the specimen as described above, the number of first cells contained in a measurement specimen to be supplied to the subsequent detection step, for example, can be made close to the number of first cells contained in the specimen before separation. Thus, for example, in the case where the first cells are rare cells, and the state of the disease is judged based on the detection result of the first cells, the first cells are properly separated from a specimen before separation, and the detection is performed on a measurement specimen containing enough first cells. This process prevents a false negative in judging the state of the disease, which is caused because the rare cells are lost in separation, and as a result, the number of rare cells contained in the specimen after the separation is small.

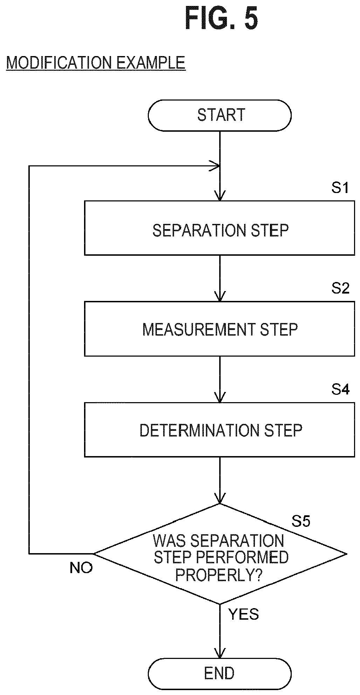

Note that the method of determining whether a separation is successful illustrated in FIG. 1 may be performed as illustrated in FIG. 5.

FIG. 5 is a flowchart illustrating the steps of a method of determining whether a separation is successful, according to a modification example of an embodiment.

In the modification example of the method of determining whether a separation is successful, step S4 is added instead of step S3, and step S5 is added after step S4, as compared to FIG. 1.

In the determination step at step S4, the particle separation apparatus determines whether the separation step is successful based on the first information and the second information in the same way as described above. In the determination step at step S4, the particle separation apparatus further determines whether the separation step is successful based on the sum of the first information and the second information. Specifically, the particle separation apparatus determines whether the separation step is successful by determining whether the sum of the first information and the second information is larger than a specified threshold. Thus, in the determination step at step S4, two determinations are performed concerning the separation step.

Meanwhile, in the case where a failure has occurred in the chip 100, the pump 104a which feeds a specimen to the chip 100, or the like in the separation step, it is possible that amounts concerning the first specimen and second specimen obtained are decreased. In this case, since appropriate amounts of the first specimen and second specimen are not obtained, the separation step is not performed correctly. Thus, by determining, in the determination step at step S4, whether the sum of the first information on an amount of the first specimen and the second information on an amount of the second specimen is larger than a specified threshold, it is possible to determine further accurately whether the separation step is successful.

At step S5, the particle separation apparatus determines whether the separation step has been properly performed based on the two determination results on the separation step obtained at step S4. The particle separation apparatus determines that the separation step has been performed properly in the case where: the particle separation apparatus determines that the separation step has been correctly performed based on the first information and the second information; and the particle separation apparatus determines the sum of the first information and the second information is larger than a specified threshold. In this case, the process in FIG. 5 ends.

On the other hand, in the case where the particle separation apparatus has determined in at least one determination of the two determinations that the separation step has not been performed correctly, the particle separation apparatus returns the process to step S1. In this case, if the specimen in the second state that can be supplied to the chip 100 remains, the particle separation apparatus performs the process after step S1 again, using the specimen in the second state. If the specimen in the second state that can be supplied to the chip 100 does not remain, the operator collects blood again from the subject and refines a specimen in the second state. Then, the particle separation apparatus performs again the process on and after step S1, using the refined specimen in the second state. Here, the first specimen obtained in the separation step may be supplied again to the separation step.

In the case that it is determined at step S5 that the separation step has not been properly performed, the separation step is performed again as described above, and it prevents an improper first specimen from being supplied to the subsequent process and thus makes it possible to resume the separation step smoothly to obtain a proper first specimen.

<Study in Method of Determining Whether a separation is Successful>

Next, description is made of the study that the inventors have conducted on methods of determining whether a separation is successful. This study includes the following steps 1 to 6.

1. Preparation of Specimen

CTCs obtained from a CTC model cell line are stained with a specified reagent. Then, the CTCs are added to PBS such that several to several hundred CTCs are contained in the PBS, and thus a specimen corresponding to the second state in FIG. 2 is prepared. Counting CTCs is visually performed with a microscope. Twelve kinds of such specimens are prepared.

2. Separation of Specimen

Each of the twelve kinds of specimens prepared at step 1 is supplied to a particle separation apparatus, and the specimen is separated to a first specimen and a second specimen using a chip 100 illustrated in FIG. 3A in the particle separation apparatus. Here, when several specimens of the twelve kinds of specimens are separated, the installation position of a container for storing the second specimen is vertically varied in the particle separation apparatus so that the pressure inside the micro flow path 110 of the chip 100 varies, and thereby failures are intentionally caused in the separation step.

3. Measurement of Weight of First Specimen and Second Specimen

For each of the twelve kinds of specimens, the weight of the first specimen and the second specimen separated at step 2 is measured with an electronic balance. Hence, in this study, the first information is the weight of the first specimen, and the second information is the weight of the second specimen.

4. Calculation of Ratio

Here, the ratio based on the first information and the second information is set to (the first information + the second information)/the first information, and the ratio is calculated for each of the twelve kinds of specimens.

5. Calculation of Recovery Ratio

For each of the twelve kinds of specimens, the CTCs contained in the first specimen are visually counted with a microscope, and then a recovery ratio is calculated by dividing the number of CTCs contained in the first specimen by the number of CTCs contained in the specimen obtained at the above step 1.

6. Drawing of Scatter Plot and Calculation of Linear Regression Equation and Coefficient of Determination

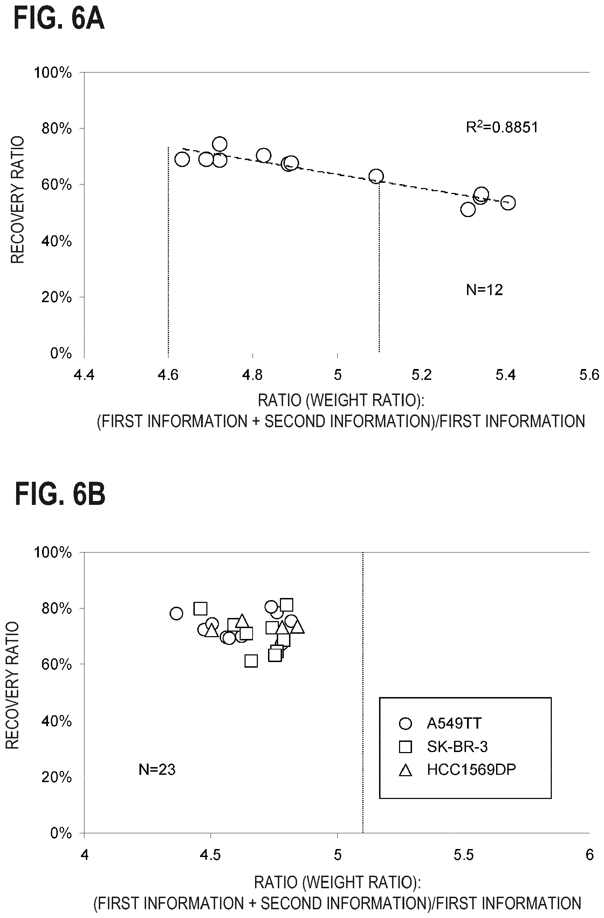

FIG. 6A is a scatter plot that is drawn in the study of the method of determining whether a separation is successful. As illustrated in FIG. 6A, the scatter plot drawn illustrates the relationship between the ratio obtained at step 4 and the recovery ratio obtained at step 5. In the scatter plot, the dot indicating the ratio and the recovery ratio is plotted for each of the twelve kinds of specimens. In the scatter plot of FIG. 6A, the recovery ratio is low for several specimens because a failure is intentionally caused in the separation step when several specimens are separated as described above. On the basis of the drawn scatter plot, the linear regression equation and the coefficient of determination are calculated. The slope of the linear regression equation is -0.25, and the coefficient of determination is 0.8851.

Since the coefficient of determination is a value close to 1 in the scatter plot drawn through above steps 1 to 6, it can be said that the dots plotted for the twelve kinds of specimens are lined, by and large, along the linear regression equation. The inventors, as above, have found that there is a correlation between the ratio and the recovery ratio.

The inventors have further found, from the fact that there is a correlation between the ratio and the recovery ratio, that the accuracy in separation of a specimen can be evaluated based on the ratio. For example, in the case of the example illustrated in FIG. 6A, it can be seen that to achieve a recovery ratio of approximately 70% or more, the ratio should preferably be at least 6.0 or less. It also can be seen that to achieve a recovery ratio of 70% in the range of CV 10%, the ratio should preferably be 5.1 or less. Thus, in the above determination step, whether the separation step is successful can be determined based on whether the ratio is lower than a specified threshold. If it is determined that the ratio is lower than the specified threshold, it can be determined that the separation step has been performed correctly.

In the case of the example illustrated in FIG. 6A, to achieve a recovery ratio of approximately 70%, it can be seen that the ratio should preferably be at least in the range of 4.0 to 6.0. It also can be seen that to achieve a recovery ratio of 70% in the range of CV 10%, the ratio should preferably be at least in the range of 4.6 to 5.1. Thus, in the above determination step, in the case where when it is determined whether the ratio is within a specified range, and where it is determined that the ratio is within the specified range, it can be determined that the separation step has been performed correctly. Here, if the recovery ratio is too high, there is a possibility that the first specimen contains a large number of unnecessary cells. However, since it is determined whether the ratio is within a specified range, when the first specimen contains a large number of unnecessary cells, it can be properly determined that the separation step has not been performed correctly.

Note that in the case where the ratio based on the first information and the second information is the first information/(the first information + the second information), whether the separation step is successful can be determined in the above determination step based on whether the ratio is higher than a specified threshold. In the case where the ratio is the first information/(the first information + the second information), the range and thresholds to be used for determination are different from the values explained with reference to FIG. 6A. Thus, the range and thresholds to be used for determination are set depending on the calculation formula of the ratio.

Although the first information and the second information are the weight in this study, in the case where the first information and the second information are the flow speed as described above, the range and thresholds to be used for determination are different from those used in the case where the first information and the second information are the weight. Specifically, the weight is the product of the flow speed, the cross-sectional area, the period of time for which the specimen has flowed, and the specific gravity of the specimen. Hence, in the case where the values of specified parameters of the parameters that are multiplied to calculate the weight are reflected in advance on the range and thresholds to be used for determination, the range and thresholds to be used for determination are different from the values explained with reference to FIG. 6A. Hence, the range and thresholds used for determination are set depending on the kind of the first information and the second information.

Verification of Method of Determining Whether a separation is Successful>

Next, description is made of verification of the method of determining whether a separation is successful, conducted by the inventors. This verification includes the following steps 1 to 6.

1. Preparation of Specimen

Cancer cells obtained from cancer cell lines are stained with a specified reagent. Then, the cancer cells are added to blood collected from a healthy human such that several to several hundred cancer cells are contained in the blood, and a specimen corresponding to the first state in FIG. 2 is prepared. Counting cancer cells is visually performed with a microscope. Ninety-five kinds of such specimens are prepared. The used cancer cell lines are the lung cancer cell line "A549TT", the breast cancer cell line "SK-BR-3", and the breast cancer cell line "HCC1569DP".

2. Separation of Specimen

Each of the 95 kinds of specimens prepared at step 1 is supplied to a particle separation apparatus, and the specimen is separated to a first specimen and a second specimen using a chip 100 illustrated in FIG. 3A in the particle separation apparatus

3. Measurement of Weight of First Specimen and Second Specimen

For each of the 95 kinds of specimens, the weight of the first specimen and the second specimen separated at step 2 is measured with an electronic balance. Hence, in this study, the first information is the weight of the first specimen, and the second information is the weight of the second specimen.

4. Calculation of Ratio

Here, the ratio based on the first information and the second information is set to (the first information + the second information)/the first information, and the ratio is calculated for each of the 95 kinds of specimens.

5. Calculation of Recovery Ratio

For each of the 95 kinds of specimens, the cancer cells contained in the first specimen are visually counted with a microscope, and then the number of cancer cells contained in the first specimen is divided by the number of cancer cells contained in the specimen obtained at the above step 1 to calculate the recovery ratio.

6. Drawing of Scatter Plot

FIG. 6B is a scatter plot drawn in the verification of the method of determining whether a separation is successful. As illustrated in FIG. 6B, the scatter plot drawn illustrates the relationship between the ratio obtained at step 4 and the recovery ratio obtained at step 5. In the scatter plot, the dot indicating the ratio and the recovery ratio is plotted for each of 23 kinds of specimens that achieved a recovery ratio of 70% in the range of CV 10%, out of the 95 kinds of specimens.

In the scatter plot drawn through the above steps 1 to 6, the threshold 5.1, which has been obtained in the above study of the method of determining whether a separation is successful, is set. In this case, the ratios of all the specimens are lower than the threshold, as illustrated in FIG. 6B. Hence, it can be seen that the threshold set for the ratio makes it possible to determine without an error that the separation step has been performed correctly for the 23 kinds of specimens for which the first cells have been properly recovered. This result suggests that whether the separation step is successful can be determined based on specimens actually collected from subjects in clinical laboratory test.

<Particle Detection Method>

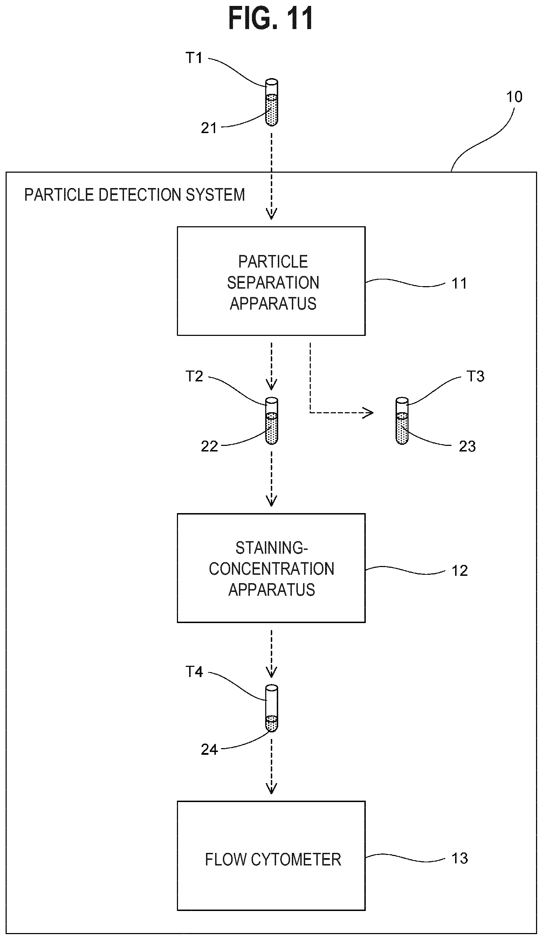

Next, a particle detection method is described.

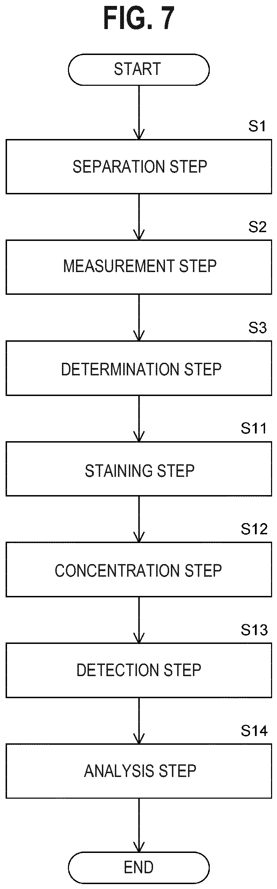

FIG. 7 is a flowchart illustrating the steps of a particle detection method.
The particle detection method includes steps S1 to S3, which are the same as or similar to those in FIG. 1, and steps S11 to S14. Hereinafter, description is made of the case that an operator inputs an instruction to apparatuses of a particle detection system in each step, and the apparatuses of the particle detection system execute each step according to the inputted instruction. Note that all the steps in steps S1 to S3 and S11 to S14 may be performed automatically by the particle detection system or may be performed by the operator operating the instruments. The particle detection system is described later with reference to FIG. 11 and subsequent figures.
Since steps S1 to S3 are the same as or similar to the processes described with reference to FIG. 1, step S11 to S14 are described in the following. Supplied to steps S11 to S14 is a first specimen for the case that the separation of the specimen has been determined to have been performed properly in the determination step at step 3.
In the staining step at step S11, the particle detection system stains the first cells contained in the first specimen for detection with a flow cytometer.

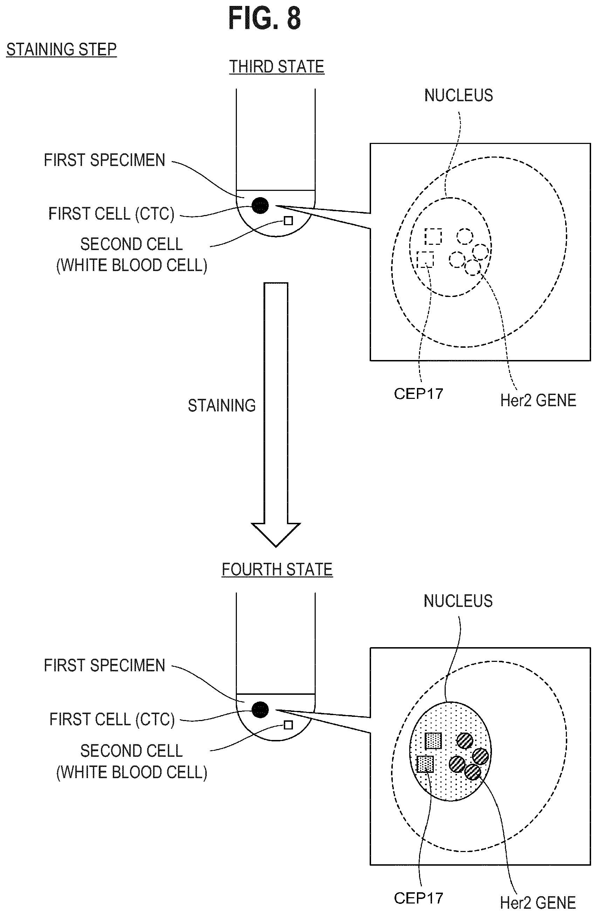

FIG. 8 is a schematic diagram for explaining the staining step at step S11.
At step S11, the particle detection system mixes a reagent for staining into the first specimen in the third state to stain the target sites of the first cells. Specifically, the target sites are fluorescently labeled with fluorescent dyes. In an embodiment, the target sites that are stained are the nucleus, Her2 genes, and the centromere regions (CEP17) of chromosome 17. As illustrated in the upper diagram of FIG. 8, any of the nucleus, Her2 genes, and CEP17 have not been stained in the third state. By performing the staining step at step S11, the nucleus, Her2 genes, and CEP17 are stained with different fluorescent dyes as illustrated in the lower diagram of FIG. 8. In this way, the state of the first specimen is changed from the third state to the fourth state.
Note that in the above staining step, the target sites to be stained may by any site in the cell. The target sites to be stained are not limited to the genes and the nucleus, but may be, for example, the cell protein, the cytoplasm, the cell membrane, the surface antigens on the cell membrane. The method of staining the target sites is not limited to a method based on in-situ hybridization and specific staining, but the target sites may be stained by immunostaining based on antigen-antibody reaction. In the case where the target sites are genes, the target sites are not limited to the Her2 gene and CEP17 but may be other gene regions. In an embodiment, cell staining means staining genes, the nucleus, proteins, the cytoplasm, the cell membrane, surface antigens, and the like of the cell, as described above.
In the above staining step, instead of staining genes and nucleus of the cell, protein and cytoplasm of the cell may be stained. Alternatively, along with staining genes and the nucleus of the cell, proteins and the cytoplasm of the cell may be stained. In the case of staining proteins in the cytoplasm, the cytoplasm should preferably be stained. Examples of proteins in the cytoplasm include PSA in CTCs originated from prostate cancer and cytokeratin in CTCs originated from lung cancer.
Note that in the case where the first cells can be detected, without the staining step, in the detection step at the subsequent step S13, the staining step may be omitted.
Returning to FIG. 7, in the concentration step at step S12, the particle detection system obtains a measurement specimen in which the concentration of the first cells is higher than the one in the first specimen.

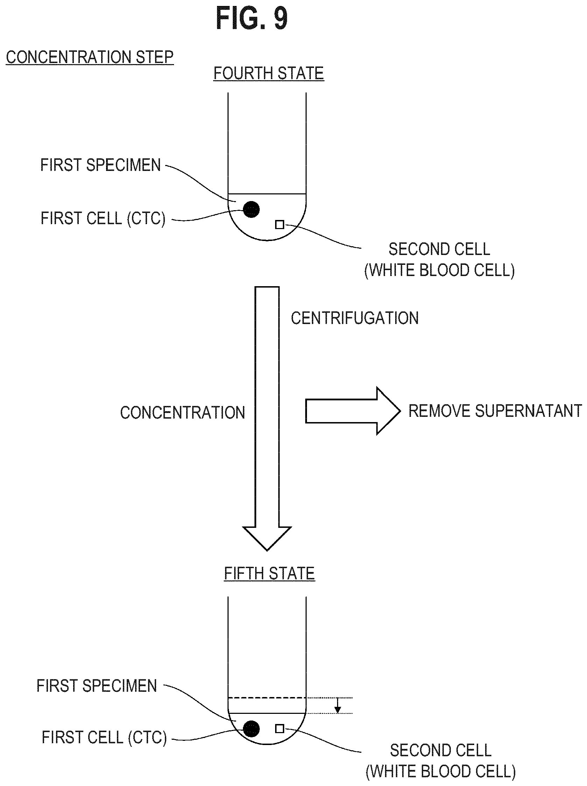

FIG. 9 is a schematic diagram for explaining the concentration step at step S12.

At step S12, the particle detection system performs processes such as centrifugation on the first specimen in the fourth state and then removes the supernatant of the first specimen after the centrifugation. With this operation, the liquid volume decreases in the state where the cells contained in the first specimen in the fourth state remain, and thus the concentration of the first cells increases. In this way, the first specimen in the fourth state becomes a measurement specimen in the fifth state. In the concentration step, the first specimen is concentrated such that the concentration of the cells contained in the measurement specimen is a specified concentration.

The concentration step, in which the concentration of the cells contained in the measurement specimen becomes a specified one, enables detection with a flow cytometer to be performed properly and efficiently in the subsequent detection step.

Returning to FIG. 7, in the detection step at step S13, the particle detection system supplies the measurement specimen obtained in the concentration step to a flow cytometer to detect the first cells.

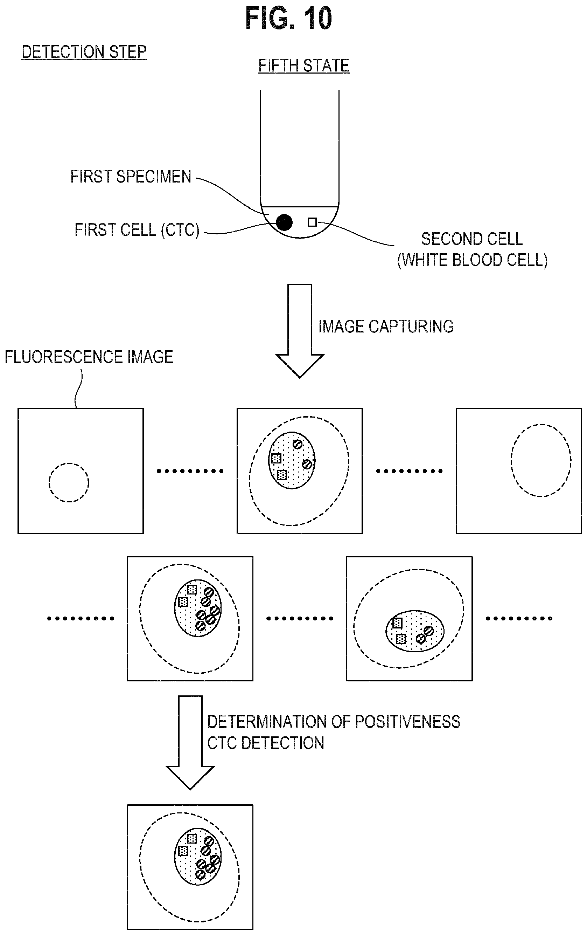

FIG. 10 is a schematic diagram for explaining the detection step at step S13.

At step S13, the particle detection system supplies the measurement specimen in the fifth state to the flow cytometer and captures images of fluorescence generated from cells contained in the measurement specimen with an image capturing unit provided in the flow cytometer. With this operation, a fluorescence image is captured for each cell contained in the measurement specimen. In addition, the flow cytometer determines whether Her2 genes have been amplified based on the obtained fluorescence images, and detects the positive cells in which the Her2 genes have been amplified as CTCs.

Note that in the detection step, the CTC detection may be performed with a microscope instead of a flow cytometer. In this case, for example, an operator uses a microscope and visually detects the positive cells in which the Her2 genes have been amplified.

As described above, supplied to steps S11 to S14 is a first specimen for the case that the separation of the specimen has been determined to have been performed properly in the determination step at step 3. Then, at step S13, the first cells are detected with the flow cytometer based on the first specimen for the case that the separation of the specimen has been determined to have been performed properly in the determination step. With this operation, the number of the first cells contained in the measurement specimen supplied to the flow cytometer can be made close to the number of the first cells contained in the specimen before separation. Thus, for example, in the case where the first cells are rare cells and where the state of a disease is judged based on the detection result of the first cells, the detection is performed on a measurement specimen that has been properly separated from a specimen before separation and contains enough first cells. Thus, it is possible to prevent a false negative in judgment of the state of the disease caused because the number of rare cells contained in the specimen after the separation is small.

Returning to FIG. 7, in the analysis step at step S14, the particle detection system analyzes the first cells detected in the detection step. Specifically, the flow cytometer, which detected the first cells, obtains the number of the first cells, the ratio of the number of the first cells to the number of all the cells captured by the image capturing unit, and other information.

Note that in the case where the first cells are circulating endothelial cells (CECs), it is analyzed in the analysis step whether $NF_KB$, which is a protein contained in the CEC, is localized in the cell. Specifically, in the staining step, the CECs are fluorescently labeled via CD146 labeled antibodies which specifically bind to antibodies expressed in the CECs, and $NF_KB$ is fluorescently labeled via labeled antibodies that specifically bind to $NF_KB$. In the detection step, images of fluorescence based on CECs are captured, and images of fluorescence based on $NF_KB$ are captured. Then, CECs are detected based on the fluorescence images. In the analysis step, it is determined whether $NF_KB$, which is signal molecules, is localized in the nucleus, and thereby it is determined whether the CEC is activated.

In the case where the first cells are CTCs originated from lung cancer and where the target site is cytokeratin, the first cells are detected in the detection step based on the amount of cytokeratin in the cytoplasm. Then, in the analysis step, the number of first cells and the rate of the first cells are obtained.

In the case where the first particles are exosomes, in the analysis step, mRNA, miRNA, or protein contained in the exosome or lipid, protein, or the like contained in the exosome membrane is analyzed. In the case where the first particles are particles contained in plasma, plasma is obtained as a first specimen in the separation step, and on the basis of the plasma obtained in the separation step, measurement on coagulation testing or measurement on immune testing is performed in the detection step. The measurement on coagulation testing is performed using a coagulation measurement apparatus, and the measurement on immune testing is performed using an immune measurement apparatus. Then, in the analysis step, the plasma is analyzed based on the measurement results.

<Particle Detection System>

Next, description is made of a particle detection system 10 for performing steps S1 to S3 and S11 to S14 in FIG. 7.

FIG. 11 is a schematic diagram illustrating the configuration of the particle detection system 10. The particle detection system 10 includes a particle separation apparatus 11, a staining-concentration apparatus 12, and a flow cytometer 13.

The particle separation apparatus 11 performs the separation step at step S1, the measurement step at step S2, and the determination step at step S3, using a specimen 21 in the second state stored in a container T1. Then, the particle separation apparatus 11 obtains a first specimen 22 stored in a container T2 and a second specimen 23 stored in a container T3. The staining-concentration apparatus 12 performs the staining step at step S11 and the concentration step at step S12 using the first specimen 22 stored in the container T2. Then, the staining-concentration apparatus 12 obtains a measurement specimen 24 stored in a container T4. The flow cytometer 13 performs the detection step at step S13 and the analysis step at step S14 using the measurement specimen 24 stored in the container T4.

Note that although the particle detection system 10 is configured including multiple apparatuses but may be configured with one apparatus.

FIG. 12 is a schematic diagram illustrating the configuration of the particle separation apparatus 11. The particle separation apparatus 11 includes a controller 101, memory 102, display-input unit 103, separation unit 104, and measurement unit 105.

The controller 101 includes a CPU. The controller 101 performs various processes based on programs stored in the memory 102. The controller 101 is connected to other units in the particle separation apparatus 11, and the controller 101 receives signals from those units and controls the units. The memory 102 includes RAM, ROM, a hard disk, or the like. The display-input unit 103 includes a touch panel display. Note that instead of the display-input unit 103, the particle separation apparatus 11 may include a display unit including a liquid crystal display and an input unit including a mouth and a keyboard.

The specimen 21 in the second state supplied to the particle separation apparatus 11 is transferred to the separation unit 104. The separation unit 104 includes a pump 104a for applying positive pressure to the input end portion 120 of the chip 100. For every separation operation of a specimen 21, the operator replaces the chip 100 with a new chip 100. Specifically, the used chip 100 is removed, and then the unused chip 100 is set to the separation unit 104. The chip 100 is a replaceable part. The separation unit 104 feeds the specimen 21 in the second state into the inlet 121 and feeds sheath liquid into the inlet 122. At this time, the separation unit 104 drives the pump 104a to apply positive pressure to the inlets 121 and 122. This determines the speed of the specimen 21 flowing in the micro flow path 110, and the specimen 21 is separated to the first specimen 22 and the second specimen 23.

The micro flow path 110 is formed in the chip 100 that is replaceable. This configuration makes it possible to separate a different specimen 21 by just replacing the chip 100 without troublesome works, such as cleaning the micro flow path 110 for every separation operation of a specimen 21. The separation unit 104 separates the specimen 21 to the first specimen 22 and the second specimen 23 by running the specimen 21 in the micro flow path 110. This makes it possible to downsize the configuration for separating the specimen 21.

As explained with reference to FIG. 4B, the outlets 131 and 132 of the chip 100 are connected to the first flow path 141 and the second flow path 142, respectively. The measurement unit 105 includes the pressure detector 151 provided in the first flow path 141, the pressure detector 152 provided in the second flow path 142, and the calculation unit 153 that calculates flow speeds. The measurement unit 105 makes the pressure detectors 151 and 152 to detect pressure and makes the calculation unit 153 calculate flow speeds based on detection signals of the pressure detectors 151 and 152. With these operations, the measurement unit 105 obtains the first information and the second information.

The controller 101 determines whether the separation of the specimen 21 by the separation unit 104 is successful based on the first information and the second information measured in the measurement unit 105. Then, the controller 101 makes the display-input unit 103 display the determination result on the specimen 21.

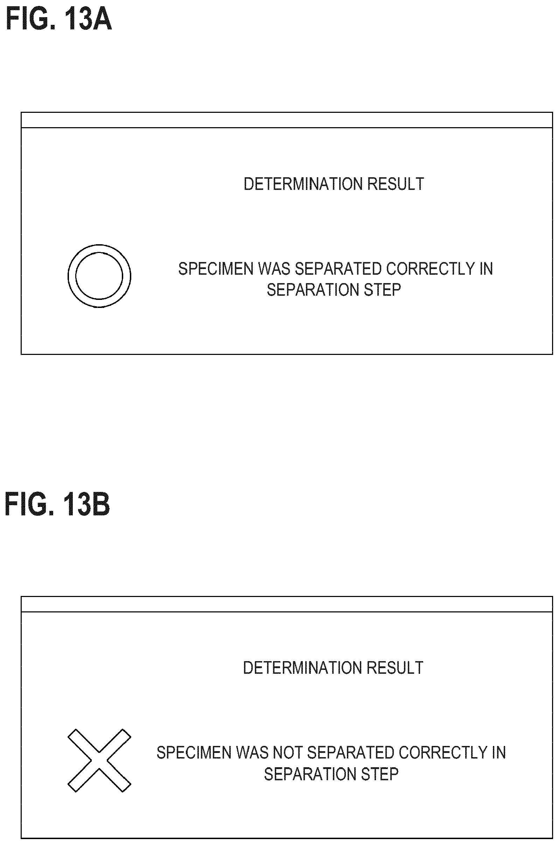

FIGs. 13A and 13B are schematic diagrams illustrating screens including determination results, displayed on the display-input unit 103 of the particle separation apparatus 11.

In the case where the controller 101 has determined that the separation of the specimen 21 has been performed correctly, the controller 101 makes the display-input unit 103 display a screen including a message indicating that the separation of the specimen 21 has been performed correctly in the separation step, as illustrated in FIG. 13A. On the other hand, in the case where the controller 101 has determined that the separation of the specimen 21 has not been performed correctly, the controller 101 makes the display-input unit 103 display a screen including a message indicating that the separation of specimen 21 has not been performed correctly in the separation step, as illustrated in FIG. 13B.

With this operation, the operator can visually realize whether the separation of the specimen 21 has been performed properly. In the case where the operator has realized that the separation has not been performed properly, the operator can prevent the first specimen 22 obtained in the improper separation from being supplied to the subsequent apparatus.

Returning to FIG. 12, the first specimen 22 that exited from the outlet 131 and passed through the first flow path 141 is stored in the container T2. The second specimen 23 that exited from the outlet 132 and passed through the second flow path 142 is stored in the container T3. The second specimen 23 in an embodiment is discarded because it is not used in the subsequent process. Thus, the first specimen 22 in the third state is obtained, and the process of the particle separation apparatus 11 ends.

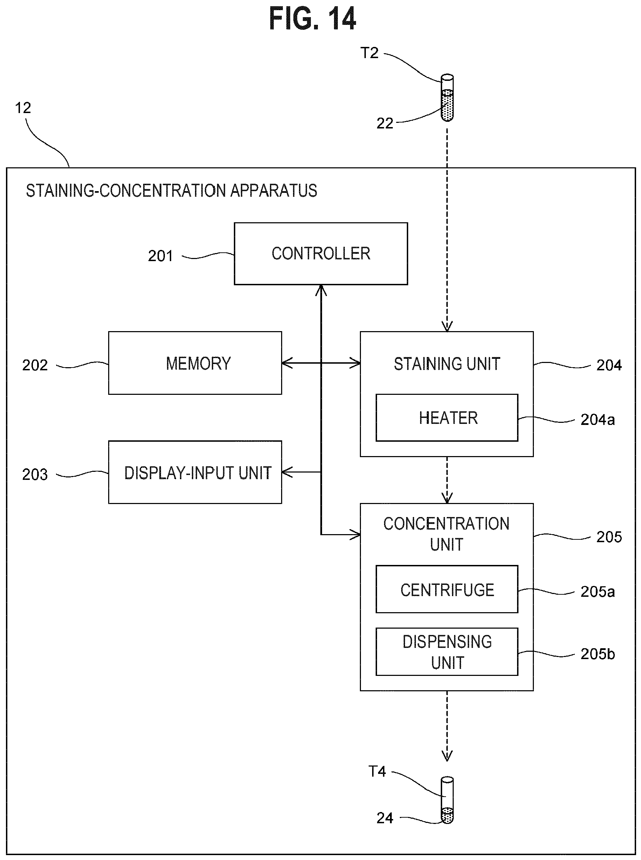

FIG. 14 is a schematic diagram illustrating the configuration of the staining-concentration apparatus 12.

The staining-concentration apparatus 12 includes a controller 201, memory 202, display-input unit 203, staining unit 204, and concentration unit 205.

The controller 201 include a CPU. The controller 201 performs various processes based on programs stored in the memory 202. The controller 201 is connected to other units in the staining-concentration apparatus 12, and the controller 201 receives signals from those units and controls the units. The memory 202 includes RAM, ROM, a hard disk, or the like. The display-input unit 203 includes a touch panel display.

The staining unit 204 includes a heater 204a. The staining unit 204 stains the first specimen 22 in the third state and obtains the first specimen 22 in the fourth state, as explained with reference to FIG. 8. The first specimen 22 in the fourth state obtained in the staining unit 204 is transferred to the concentration unit 205. The concentration unit 205 includes a centrifuge 205a and a dispensing unit 205b. The concentration unit 205 dispenses the first specimen 22 in the fourth state into a specified container and centrifuges the first specimen 22 in the fourth state with the centrifuge 205a. Then, the concentration unit 205 removes the centrifuged supernatant in the container with the dispensing unit 205b. With these operations, the first specimen 22 in the fourth state is concentrated as explained with reference to FIG. 9. The concentration unit 205 transfers the first specimen 22 from which the supernatant has been removed to the container T4 as a measurement specimen 24. In this way, the measurement specimen 24 in the fifth state is obtained, and the process of the staining-concentration apparatus 12 ends.

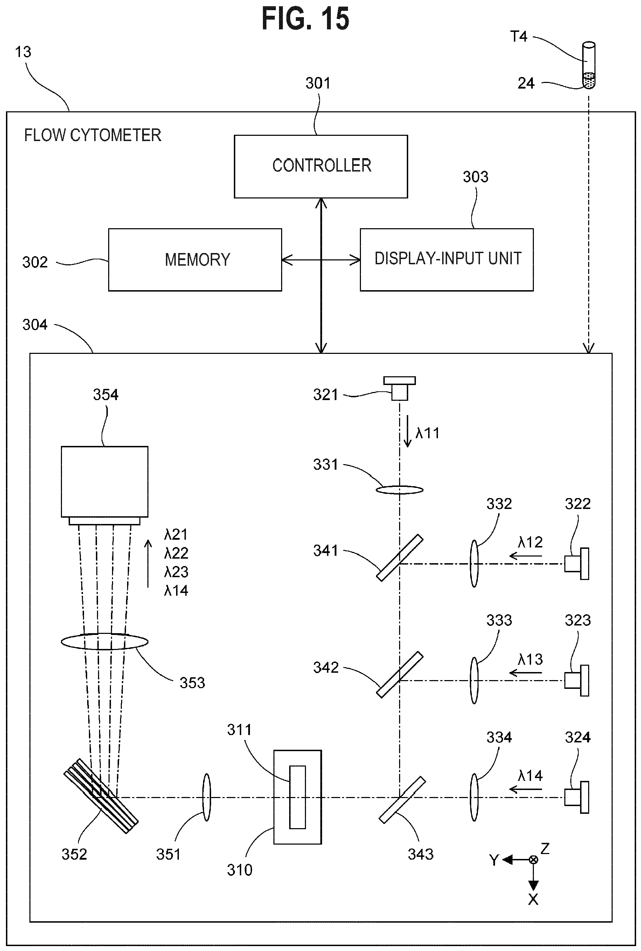

FIG. 15 is a schematic diagram illustrating the configuration of the flow cytometer 13. The flow cytometer 13 includes a controller 301, memory 302, display-input unit 303, and measurement unit 304. In addition, the flow cytometer 13 also includes an aspiration tube for aspirating the measurement specimen 24 in the container T4, a flow path for transferring the aspirated measurement specimen 24 to a flow cell 310, and a flow path for collecting the measurement specimen 24 discharged from the flow cell 310.

The controller 301 include a CPU. The controller 301 performs various processes based on programs stored in the memory 302. The controller 301 is connected to other units in the flow cytometer 13, and the controller 301 receives signals from those units and controls the units. The memory 302 includes RAM, ROM, a hard disk, or the like. The display-input unit 303 includes a touch panel display.

The measurement unit 304 includes the flow cell 310, light sources 321 to 324, condenser lenses 331 to 334, dichroic mirrors 341 to 343, a condenser lens 351, an optical unit 352, a condenser lens 353, and an image capturing unit 354. The measurement unit 304 projects light to the measurement specimen 24 containing multiple kinds of fluorescently labeled target sites and detects multiple kinds of fluorescence with different wavelengths. In the measurement unit 304 in FIG. 15, XYZ axes orthogonal to one another are indicated.

The measurement specimen 24 flows in a flow path 311 of the flow cell 310 in the positive direction of the Z axis. The light sources 321 to 324 project light to the measurement specimen 24 flowing in the flow cell 310. The light sources 321 to 324 each include a semiconductor laser light source. The light beams emitted from the light sources 321 to 324 are laser light beams with wavelengths of $\lambda 11$ to $\lambda 14$, respectively. The condenser lenses 331 to 334 respectively collect the light beams emitted from the light sources 321 to 324. The dichroic mirror 341 passes light with a wavelength of $\lambda 11$ and reflects light with a wavelength of $\lambda 12$. The dichroic mirror 342 passes light with wavelengths of $\lambda 11$ and $\lambda 12$ and reflects light with a wavelength of $\lambda 13$. The dichroic mirror 343 reflects light with wavelengths of $\lambda 11$ to $\lambda 13$ and passes light with a wavelength of $\lambda 14$. In this way, light with wavelengths of $\lambda 11$ to $\lambda 14$ is projected in the positive direction of the Y axis to the measurement specimen 24 flowing in the flow path 311 of the flow cell 310.

Meanwhile, the fluorescent dyes respectively used for labeling the nucleus and the two genes are selected out of: a fluorescent dye that generates fluorescence with a wavelength of $\lambda 21$ when it receives excitation light with a wavelength of $\lambda 11$; a fluorescent dye that generates fluorescence with a wavelength of $\lambda 22$ when it receives excitation light with a wavelength of $\lambda 12$; and a fluorescent dye that generates fluorescence with a wavelength of $\lambda 23$ when it receives excitation light with a wavelength of $\lambda 13$. An Image of fluorescence with each of the wavelengths of $\lambda 21$ to $\lambda 23$ is captured, and thereby fluorescence images can be obtained of the nucleus and the two genes, which are target sites.

When light with wavelengths of $\lambda 11$ to $\lambda 14$ is projected to the measurement specimen 24 flowing in the flow cell 310, fluorescence is generated from the fluorescent dyes used for labeling the target sites of CTCs. When light with a wavelength of $\lambda 14$ is projected to the measurement specimen 24 flowing in the flow cell 310, this light passes through the cells. The light with a wavelength of $\lambda 14$ having passed through the cells is used to produce a bright-field image.

The condenser lens 351 condenses fluorescence with wavelengths of $\lambda 21$ to $\lambda 23$ emitted from the measurement specimen 24 and light with a wavelength of $\lambda 14$ having passed through the measurement specimen 24. The optical unit 352 includes a combination of four dichroic mirrors. The four dichroic mirrors of the optical unit 352 reflect the fluorescence with wavelengths of $\lambda 21$ to $\lambda 23$ and the light with a wavelength of $\lambda 14$ at angles slightly different from one another and separate them on the light receiving surface of the image capturing unit 354. The condenser lens 353 condenses the fluorescence with wavelengths of $\lambda 21$ to $\lambda 23$ and the light with a wavelength of $\lambda 14$.

The image capturing unit 354 includes a time delay integration (TDI) camera. The image capturing unit 354 captures images of the fluorescence with wavelengths of λ21 to λ23 and the light with a wavelength of λ14 and produces fluorescence images respectively corresponding to the fluorescence with the wavelengths of λ21 to λ23 and the bright-field image corresponding to the light with a wavelength of λ14. The controller 301 stores the fluorescence images and the bright-field image produced by the image capturing unit 354 into the memory 302.

Here, since the image capturing unit 354 includes a TDI camera, the fluorescence images and the bright-field image are produced by integrating the fluorescence received on the light receiving surface of the image capturing unit 354. This configuration improves the quality of the fluorescence images of cells and the bright-field image.

The controller 301 determines whether each cell is positive and detects CTCs based on the fluorescence images stored in the memory 302 as explained with reference to FIG. 10. Specifically, the controller 301 extracts or finds bright spots on the fluorescence image produced by the fluorescent dye for labeling Her2 genes and the fluorescence image produced by the fluorescent dye for labeling CEP17. The controller 301 divides the number of bright spots based on Her2 genes by the number of bright spots based on CEP17 for each cell, and if the calculated value is larger than 1 in a cell, the controller 301 determines that the Her2 gene is amplified in the cell. Then, the controller 301 detects positive cells in which the Her2 gene has been amplified, as CTCs. Then, the controller 301 obtains the number of detected CTCs, the ratio of the number of CTCs to the number of all the cells captured by the image capturing unit 354, and other information.

Note that the flow cytometer 13 in an embodiment is what is called an imaging flow cytometer which detects cells based on captured images, but it is not limited to this type. The flow cytometer 13 may be a flow cytometer that detects light generated from cells with a photodetector and detects cells based on detection signals of the photodetector.

Furthermore, in an embodiment, in the case where the controller 101 has determined that the separation of the specimen 21 has been performed correctly, the controller 101 makes the display-input unit 103 display a screen including a message indicating that the separation of the specimen 21 has been performed correctly in the separation step, as illustrated in FIG. 13A. However, the invention is not limited to this embodiment. Even in the case where the controller has determined that the separation of the specimen has been performed correctly, the controller 101 may not output information indicating that the separation of the specimen has been performed correctly. The controller may output information such as an error or alert indicating that the separation of the specimen has not been performed correctly only in the case where the controller has determined that the separation of the specimen has not been performed correctly.

The invention includes other embodiments in addition to the above-described embodiments without departing from the spirit of the invention. The embodiments are to be considered in all respects as illustrative, and not restrictive. The scope of the invention is indicated by the appended claims rather than by the foregoing description. Hence, all configurations including the meaning and range within equivalent arrangements of the claims are intended to be embraced in the invention.

## Claims

1. A method of determining a quality of a cell separation, comprising:

   separating, by running a specimen containing a first particle and a second particle in a flow path, the specimen to a first specimen containing the first particle and a second specimen containing the second particle, based on a difference in magnitude of a force exerted on each particle contained in the specimen;
   measuring first information on an amount of the first specimen and second information on an amount of the second specimen; and
   determining a quality of the separating based on the first information and the second information.

2. The method according to claim 1, wherein the separating comprises separating the specimen to:

   the first specimen in which a presence ratio of the first particle is higher than in the specimen; and
   the second specimen in which a presence ratio of the first particle is lower than in the specimen.

3. The method according to claim 1 or 2, wherein the separating comprising running the specimen containing a sample and sheath liquid in the flow path, and
   the first specimen and the second specimen contain the sample and the sheath liquid.

4. The method according to any one of claims 1 to 3, wherein
   the measuring comprises measuring the first information and the second information based on a detection signal of a detector provided at each of a first flow path in which the first specimen flows and a second flow path in which the second specimen flows.

5. The method according to any one of claims 1 to 4, wherein

the first information comprises weight or volume of the first specimen, and
the second information comprises weight or volume of the second specimen.

6. The method according to any one of claims 1 to 5, wherein
the first information and the second information enable calculation of weight or volume of the first specimen and the second specimen, respectively.

7. The method according to any one of claims 1 to 6, wherein
the first information and the second information comprise information on pressure or flow speed.

8. The method according to any one of claims 1 to 7, wherein
the determining comprises determining the quality of the separating based on a ratio calculated from the first information and the second information.

9. The method according to claim 8, wherein the ratio comprises:

a ratio of a sum of the first information and the second information to the first information;
a ratio of the sum of the first information and the second information to the second information;
a ratio of the first information to the sum of the first information and the second information;
a ratio of the second information to the sum of the first information and the second information;
a ratio of the second information to the first information; or
a ratio of the first information to the second information.

10. The method according to claim 8 or 9, wherein
the determining comprises determining the quality of the separating by comparing the ratio and a specified threshold.

11. The method according to any one of claims 8 to 10, wherein
the determining comprises determining the quality of the separating based on whether the ratio is within a specified range.

12. The method according to any one of claims 1 to 11, wherein
the determining comprises determining the quality of the separating based on a sum of the first information and the second information.

13. The method according to any one of claims 1 to 12, further comprising:
in response to the determining that the separating has not been successful, performing the separating again.

14. The method according to any one of claims 1 to 13, wherein
the specimen comprises blood;
the first particle comprises a circulating tumor cell; and
the second particle comprises at least one kind of blood cell selected from a red blood cell, a white blood cell, or a platelet.

15. A particle separation apparatus comprising:

a separation unit that separates, by running a specimen containing a first particle and a second particle in a flow path, the specimen to a first specimen containing the first particle and a second specimen containing the second particle, based on a difference in magnitude of a force exerted on each particle contained in the specimen;
a measurement unit that measures first information on an amount of the first specimen and second information on an amount of the second specimen; and
a determination unit that determines a quality of the separation of the specimen by the separation unit based on the first information and the second information.

# FIG. 1

```
        START
          │
          ▼                    S1
┌─────────────────────┐
│   SEPARATION STEP    │
└─────────────────────┘
          │
          ▼                    S2
┌─────────────────────┐
│   MEASUREMENT STEP   │
└─────────────────────┘
          │
          ▼                    S3
┌─────────────────────┐
│  DETERMINATION STEP  │
└─────────────────────┘
          │
          ▼
         END
```

# FIG. 2

SEPARATION STEP

FIRST STATE

SPECIMEN (BLOOD)

SECOND CELL (RED BLOOD CELL)

SECOND CELL (PLATELET)

FIRST CELL (CTC)

SECOND CELL (WHITE BLOOD CELL)

HEMOLYZATION OF
RED BLOOD CELLS

SECOND STATE

SPECIMEN (BLOOD)

SECOND CELL (RED BLOOD CELL)

SECOND CELL (PLATELET)

FIRST CELL (CTC)

SECOND CELL (WHITE BLOOD CELL)

SEPARATION

THIRD STATE

SECOND SPECIMEN

SECOND CELL
(RED BLOOD CELL)

FIRST SPECIMEN

SECOND CELL
(PLATELET)

FIRST CELL (CTC)

SECOND CELL
(WHITE BLOOD CELL)

SECOND CELL
(WHITE BLOOD CELL)

20

## FIG. 3A

## FIG. 3B

# FIG. 4A

TO OUTLET 131

130

110

TO OUTLET 132

# FIG. 4B

MEASUREMENT STEP

130

100

131

132

141

142

153

151

152

CALCULATION
UNIT

SECOND SPECIMEN

SECOND CELL
(RED BLOOD CELL)

SECOND CELL
(PLATELET)

FIRST SPECIMEN

FIRST CELL (CTC)
SECOND CELL
(WHITE BLOOD CELL)

SECOND CELL
(WHITE BLOOD CELL)

# FIG. 5

MODIFICATION EXAMPLE

```
                    ( START )
                        │
    ┌──────────────────►│
    │                   │                      S1
    │          ┌─────────────────────┐
    │          │   SEPARATION STEP   │
    │          └─────────────────────┘
    │                   │
    │                   │                      S2
    │          ┌─────────────────────┐
    │          │  MEASUREMENT STEP   │
    │          └─────────────────────┘
    │                   │
    │                   │                      S4
    │          ┌─────────────────────┐
    │          │ DETERMINATION STEP  │
    │          └─────────────────────┘
    │                   │
    │                   ▼                      S5
    │              ╱         ╲
    │            ╱ WAS SEPARATION ╲
    └── NO ─────◄  STEP PERFORMED  ►
                 ╲   PROPERLY?   ╱
                   ╲          ╱
                       │
                      YES
                       │
                   ( END )
```

## FIG. 6A

R²=0.8851 / N=12

RECOVERY RATIO vs RATIO (WEIGHT RATIO): (FIRST INFORMATION + SECOND INFORMATION)/FIRST INFORMATION

## FIG. 6B

N=23

Legend:
- ○ A549TT
- □ SK-BR-3
- △ HCC1569DP

RATIO (WEIGHT RATIO): (FIRST INFORMATION + SECOND INFORMATION)/FIRST INFORMATION

# FIG. 7

```
        ┌─────────────┐
        │   START     │
        └──────┬──────┘
               │                    S1
        ┌──────▼──────────────┐
        │  SEPARATION STEP    │
        └──────┬──────────────┘
               │                    S2
        ┌──────▼──────────────┐
        │  MEASUREMENT STEP   │
        └──────┬──────────────┘
               │                    S3
        ┌──────▼──────────────┐
        │ DETERMINATION STEP  │
        └──────┬──────────────┘
               │                    S11
        ┌──────▼──────────────┐
        │   STAINING STEP     │
        └──────┬──────────────┘
               │                    S12
        ┌──────▼──────────────┐
        │ CONCENTRATION STEP  │
        └──────┬──────────────┘
               │                    S13
        ┌──────▼──────────────┐
        │  DETECTION STEP     │
        └──────┬──────────────┘
               │                    S14
        ┌──────▼──────────────┐
        │   ANALYSIS STEP     │
        └──────┬──────────────┘
               │
        ┌──────▼──────┐
        │    END      │
        └─────────────┘
```

# FIG. 8

STAINING STEP

THIRD STATE

NUCLEUS

FIRST SPECIMEN

FIRST CELL (CTC)

SECOND CELL
(WHITE BLOOD CELL)

CEP17          Her2 GENE

STAINING

FOURTH STATE

NUCLEUS

FIRST SPECIMEN

FIRST CELL (CTC)

SECOND CELL
(WHITE BLOOD CELL)

CEP17          Her2 GENE

26

# FIG. 9

CONCENTRATION STEP

FOURTH STATE

FIRST SPECIMEN

FIRST CELL (CTC)

SECOND CELL
(WHITE BLOOD CELL)

CENTRIFUGATION

CONCENTRATION

REMOVE SUPERNATANT

FIFTH STATE

FIRST SPECIMEN

FIRST CELL (CTC)

SECOND CELL
(WHITE BLOOD CELL)

# FIG. 10

# FIG. 11

T1

21

**PARTICLE DETECTION SYSTEM**

10

PARTICLE
SEPARATION
APPARATUS — 11

T2                              T3

22                              23

STAINING-
CONCENTRATION
APPARATUS — 12

T4

24

FLOW CYTOMETER — 13

# FIG. 12

T1

21

11

PARTICLE SEPARATION APPARATUS

101 CONTROLLER

102 MEMORY

103 DISPLAY-INPUT UNIT

SEPARATION UNIT 104

PUMP 104a

CHIP 100

FIRST SPECIMEN SECOND SPECIMEN

MEASUREMENT UNIT 105

PRESSURE DETECTOR 151

PRESSURE DETECTOR 152

CALCULATION UNIT 153

T2

22

T3

23

## FIG. 13A

DETERMINATION RESULT

SPECIMEN WAS SEPARATED CORRECTLY IN
SEPARATION STEP

## FIG. 13B

DETERMINATION RESULT

SPECIMEN WAS NOT SEPARATED CORRECTLY IN
SEPARATION STEP

# FIG. 14

EP 3 663 760 A1

# FIG. 15

# FIG. 16

Europäisches
Patentamt

European
Patent Office

Office européen
des brevets

# EUROPEAN SEARCH REPORT

Application Number

EP 19 21 0591

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/117593 A1 (TONER MEHMET [US] ET AL) 3 May 2018 (2018-05-03) <br> * paragraph [0015] * <br> * paragraph [0018] * <br> * paragraph [0159] * <br> * paragraph [0130] * <br> * paragraph [0179] * <br> * paragraph [0192] * <br> * paragraph [0195] * <br> * paragraph [0227] * <br> * paragraph [0229] * <br> * paragraph [0230] * <br> * paragraph [0239] * <br> * figures 4a-4c * <br> * figure 14 * <br> ----- | 1,2,4-15 | INV. <br> G01N33/49 <br> G01N15/02 <br> B01L3/00 <br><br> ADD. <br> G01N15/00 <br> G01N15/14 |
| X | US 2018/028940 A1 (SUNG HYUNG-JIN [KR] ET AL) 1 February 2018 (2018-02-01) <br> * figure 2 * <br> * paragraph [0057] * <br> * paragraph [0086] * <br> * paragraph [0107] * <br> * paragraph [0113] * <br> * paragraph [0114] * <br> * figures 6,7 * <br> ----- | 1-3,15 | |
| A | US 2012/277902 A1 (SHARPE JOHNATHAN CHARLES [NZ] ET AL) 1 November 2012 (2012-11-01) <br> * paragraph [0005] * <br> * paragraph [0082] * <br> ----- | 1-15 | |

**TECHNICAL FIELDS SEARCHED (IPC)**

G01N
B01L

The present search report has been drawn up for all claims

| Place of search | Date of completion of the search | Examiner |
|---|---|---|
| The Hague | 24 April 2020 | Traversa, Marzia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 21 0591

This annex lists the patent family members relating to the patent documents cited in the above-mentioned European search report.
The members are as contained in the European Patent Office EDP file on
The European Patent Office is in no way liable for these particulars which are merely given for the purpose of information.

24-04-2020

| Patent document cited in search report | | Publication date | Patent family member(s) | | Publication date |
|---|---|---|---|---|---|
| US 2018117593 | A1 | 03-05-2018 | CN | 101765762 A | 30-06-2010 |
| | | | CN | 103630470 A | 12-03-2014 |
| | | | EP | 2142279 A2 | 13-01-2010 |
| | | | EP | 2482055 A2 | 01-08-2012 |
| | | | EP | 2562531 A2 | 27-02-2013 |
| | | | HK | 1145874 A1 | 02-05-2014 |
| | | | HK | 1194817 A1 | 16-12-2016 |
| | | | JP | 5172946 B2 | 27-03-2013 |
| | | | JP | 5684224 B2 | 11-03-2015 |
| | | | JP | 2010538241 A | 09-12-2010 |
| | | | JP | 2013127468 A | 27-06-2013 |
| | | | US | 2009014360 A1 | 15-01-2009 |
| | | | US | 2012292233 A1 | 22-11-2012 |
| | | | US | 2013011210 A1 | 10-01-2013 |
| | | | US | 2014326339 A1 | 06-11-2014 |
| | | | US | 2017080425 A1 | 23-03-2017 |
| | | | US | 2018117593 A1 | 03-05-2018 |
| | | | US | 2019160465 A1 | 30-05-2019 |
| | | | WO | 2008130977 A2 | 30-10-2008 |
| US 2018028940 | A1 | 01-02-2018 | JP | 6338766 B2 | 06-06-2018 |
| | | | JP | 2018513348 A | 24-05-2018 |
| | | | KR | 20170088166 A | 01-08-2017 |
| | | | US | 2018028940 A1 | 01-02-2018 |
| | | | WO | 2017126755 A1 | 27-07-2017 |
| US 2012277902 | A1 | 01-11-2012 | EP | 2661614 A2 | 13-11-2013 |
| | | | US | 2012277902 A1 | 01-11-2012 |
| | | | WO | 2012094325 A2 | 12-07-2012 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017500006 A